(19) Europäisches Patentamt
European Patent Office
Office européen des brevets

(11) EP 1 851 217 B1

(12) **EUROPEAN PATENT SPECIFICATION**

(45) Date of publication and mention of the grant of the patent:
**11.02.2009 Bulletin 2009/07**

(21) Application number: **06707117.5**

(22) Date of filing: **21.02.2006**

(51) Int Cl.:
***C07D 471/04*** *(2006.01)* ***A61K 31/519*** *(2006.01)*
***A61P 35/00*** *(2006.01)*

(86) International application number:
**PCT/EP2006/001539**

(87) International publication number:
**WO 2006/087229 (24.08.2006 Gazette 2006/34)**

(54) **SUBSTITUTED PYRIDO[2,3-D]PYRIMIDINE DERIVATIVES USEFUL AS MEDICINES FOR THE TREATMENT OF AUTOIMMUNE DISORDERS**

ALS MEDIKAMENTE FÜR DIE BEHANDLUNG VON AUTOIMMUNERKRANKUNGEN GEEIGNETE SUBSTITUIERTE PYRIDO[2,3-D]PYRIMIDINDERIVATE

DERIVES SUBSTITUES DE PYRIDO [2,3-D] PYRIMIDINE UTILES EN TANT QUE MEDICAMENTS POUR LE TRAITEMENT DES TROUBLES AUTOIMMUNS

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR MK**

(30) Priority: **21.02.2005 GB 0503506**

(43) Date of publication of application:
**07.11.2007 Bulletin 2007/45**

(73) Proprietor: **4 AZA IP NV**
**3000 Leuven (BE)**

(72) Inventors:
- **DE JONGHE, Steven, Cesar, Alfons**
  **B-3080 Tervuren (BE)**
- **HERDEWIJN, Piet, André, Maurits, Maria**
  **B-3111 Rotselaar/Wezemaal (BE)**
- **GAO, Ling-Jie**
  **B-3360 Bierbeek (BE)**

(74) Representative: **Weiss, Wolfgang**
**Weickmann & Weickmann**
**Patentanwälte**
**Postfach 86 08 20**
**81635 München (DE)**

(56) References cited:
**EP-A- 0 265 126** **US-A- 5 223 503**

- **TAYLOR, E. C.; ET AL.: "Protection and Deprotection of Fused 2-Amino-4 (3H)-Pyrimidinones: Conversion of Pterins and 5-Deazapterins to 2,4,-Diamino Derivatives" HETEROCYCLES , 36(8), 1883-95 CODEN: HTCYAM; ISSN: 0385-5414, 1993, XP008041459**
- **TAYLOR, E. C.; ET AL.: "Convergent and Efficient Palladium-Effected Synthesis of 5,10-Dideaza-5,6,7,8-tetrahydrofolic Acid (DDATHF)" JOURNAL OF ORGANIC CHEMISTRY , 54(15), 3618-24 CODEN: JOCEAH; ISSN: 0022-3263, 1989, XP002228370**
- **KUWADA, TAKESHI ET AL: "A new synthesis of 6-substituted pyrido[2,3-d]pyrimidines" HETEROCYCLES , 57(11), 2081-2090 CODEN: HTCYAM; ISSN: 0385-5414, 2002, XP001246915**
- **DURUCASU, INCI: "Investigation of different synthetic ways for protection of 6-bromo-5-deazapterin" DOGA: TURK KIMYA DERGISI , 13 (3), 280-92 CODEN: DKSEE7; ISSN: 1010-7614, 1989, XP009066859**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to a class of novel trisubstituted pyrido(2,3-d)pyrimidine derivatives. The present invention relates to methods for their preparation, as well as to compositions, in particular pharmaceutical compositions comprising one or more of said trisubstituted pyrido(2,3-d)pyrimidine derivatives together with one or more pharmaceutically acceptable excipients. The present invention further relates to the use of said novel trisubstituted pyrido(2,3-d) pyrimidine derivatives as biologically active ingredients, more specifically for the manufacture of medicaments for the treatment of disorders and pathologic conditions such as, but not limited to, immune and auto-immune disorders, organ and cells transplant rejections, cell proliferative disorders, cardiovascular disorders and disorders of the central nervous system.

### BACKGROUND OF THE INVENTION

A huge number of pyrido(2,3-d)pyrimidine derivatives is already known in the art, some of them with biological activity. For instance, trisubstituted pyrido(2,3-d)pyrimidine derivatives with various substi-tuents on positions 2, 4 and 6 (using the standard atom numbering for the pyrido(2,3-d)pyrimidine moiety) are known with biological activities such as antibacterial and antiprotozoal activity, dihydrofolate reductase activity, antiarthritic activity, anti-mycobacterial activity, inhibition of cyclin-dependent kinases, antihypertensive activity, anti-neoplastic activity, and insecticides, e.g. from U.S. patent No. 5,223,503 and U.S. patent No. 5,547,954. Some other trisubstituted pyrido(2,3-d)pyrimidine derivatives are known without any particular usefulness such as biological activity, for instance 2-amino-4-hydroxy-6-bromo-pyrido(2,3-d)pyrimidine is known from Taylor et al. in Heteocycles (1993) 36:1885, and 2-amino-4-[1,2,4-triazolyl]-6-bromo-pyrido (2,3-d)pyrimidine is known from Durucasu in Turk. J. Chem. (1989) 13:280-292.

However there is a continuous need in the art for specific and highly therapeutically active compounds, such as, but not limited to, drugs for treating immune and autoimmune disorders, organ and cells transplant rejections, cell proliferative disorders, cardiovascular disorders and disorders of the central nervous system. In particular, there is a need in the art to provide immunosuppressive compounds and antineoplastic drugs which are active in a minor dose in order to replace existing drugs having significant side effects and/or to decrease treatment costs at the same therapeutic efficiency.

Currently used immunosuppressive, drugs include antiproliferative agents, such as methotrexate (a 2,4-diaminopyrido(3,2-d)pyrimidine derivative disclosed by U.S. Patent No. 2,512,572), azathioprine, and cyclophosphamide. Since these drugs affect mitosis and cell division, they have severe toxic effects on normal cells with high turn-over rate such as bone marrow cells and the gastrointestinal tract lining. Accordingly, marrow depression and liver damage are common side effects of these antiproliferative drugs.

Anti-inflammatory compounds used to induce immunosuppression include adrenocortical steroids such as dexamethasone and prednisolone. The common side effects observed with the use of these compounds are frequent infections, abnormal metabolism, hypertension, and diabetes.

Other immunosuppressive compounds currently used to inhibit lymphocyte activation and subsequent proliferation include cyclosporine, tacrolimus and rapamycin. Cyclosporine and its relatives are among the most commonly used immunosuppressant drugs. Cyclosporine is typically used for preventing or treating organ rejection in kidney, liver, heart, pancreas, bone marrow, and heart-lung transplants, as well as for the treatment of autoimmune and inflammatory diseases such as Crohn's disease, aplastic anemia, multiple-sclerosis, myasthenia gravis, uveitis, biliary cirrhosis, etc. However, cyclosporines suffer from a small therapeutic dose window and severe toxic effects including nephrotoxicity, hepatotoxicity, hypertension, hirsutism, cancer, and neurotoxicity.

Additionally, monoclonal antibodies with immunosuppressant properties, such as OKT3, have been used to prevent and/or treat graft rejection. Introduction of such monoclonal antibodies into a patient, as with many biological materials, induces several side-effects, such as dyspnea. Within the context of many life-threatening diseases, organ transplantation is considered a standard treatment and, in many cases, the only alternative to death. The immune response to foreign cell surface antigens on the graft, encoded by the major histo-compatibility complex (hereinafter referred as MHC) and present on all cells, generally precludes successful transplantation of tissues and organs unless the transplant tissues come from a compatible donor and the normal immune response is suppressed. Other than identical twins, the best compatibility and thus, long term rates of engraftment, are achieved using MHC identical sibling donors or MHC identical unrelated cadaver donors. However, such ideal matches are difficult to achieve. Further, with the increasing need of donor organs an increasing shortage of transplanted organs currently exists. Accordingly, xenotransplantation has emerged as an area of intensive study, but faces many hurdles with regard to rejection within the recipient organism.

The host response to an organ allograft involves a complex series of cellular interactions among T and B

lymphocytes as well as macrophages or dendritic cells that recognize and are activated by foreign antigen. Co-stimulatory factors, primarily cytokines, and specific cell-cell interactions, provided by activated accessory cells such as macrophages or dendritic cells are essential for T-cell proliferation. These macrophages and dendritic cells either directly adhere to T-cells through specific adhesion proteins or secrete cytokines that stimulate T-cells, such as IL-12 and IL-15. Accessory cell-derived co-stimulatory signals stimulate activation of interleukin-2 (IL-2) gene transcription and expression of high affinity IL-2 receptors in T-cells. IL-2 is secreted by T lymphocytes upon antigen stimulation and is required for normal immune responsiveness. IL-2 stimulates lymphoid cells to proliferate and differentiate by binding to IL-2 specific cell surface receptors (IL-2R). IL-2 also initiates helper T-cell activation of cytotoxic T-cells and stimulates secretion of interferon-γ which in turn activates cytodestructive properties of macrophages. Furthermore, IFN-γ and IL-4 are also important activators of MHC class II expression in the transplanted organ, thereby further expanding the rejection cascade by enhancing the immunogenicity of the grafted organ The current model of a T-cell mediated response suggests that T-cells are primed in the T-cell zone of secondary lymphoid organs, primarily by dendritic cells. The initial interaction requires cell to cell contact between antigen-loaded MHC molecules on antigen-presenting cells (hereinafter referred as APC) and the T-cell receptor/CD3 complex on T-cells. Engagement of the TCR/CD3 complex induces CD154 expression predominantly on CD4 T-cells that in turn activate the APC through CD40 engagement, leading to improved antigen presentation. This is caused partly by upregulation of CD80 and CD86 expression on the APC, both of which are ligands for the important CD28 co-stimulatory molecule on T-cells. However, engagement of CD40 also leads to prolonged surface expression of MHC-antigen complexes, expression of ligands for 4-1 BB and OX-40 (potent co-stimulatory molecules expressed on activated T-cells). Furthermore, CD40 engagement leads to secretion of various cytokines (e.g., IL-12, IL-15, TNF-α, IL-1, IL-6, and IL-8) and chemokines, all of which have important effects on both APC and T-cell activation and maturation. Similar mechanisms are involved in the development of auto-immune disease, such as type I diabetes. In humans and non-obese diabetic mice, insulin-dependent diabetes mellitus results from a spontaneous T-cell dependent auto-immune destruction of insulin-producing pancreatic .beta. cells that intensifies with age. The process is preceded by infiltration of the islets with mononuclear cells (insulitis), primarily composed of T lymphocytes. A delicate balance between auto-aggressive T-cells and suppressor-type immune phenomena determines whether expression of auto-immunity is limited to insulitis or not. Therapeutic strategies that target T-cells have been successful in preventing further progress of the auto-immune disease. These include neonatal thymectomy, administration of cyclosporine, and infusion of anti-pan T-cell, anti-CD4, or anti-CD25 (IL-2R) monoclonal antibodies. The aim of all rejection prevention and auto-immunity reversal strategies is to suppress the patient's immune reactivity to the antigenic tissue or agent, with a minimum of morbidity and mortality. Accordingly, a number of drugs are currently being used or investigated for their immunosuppressive properties. As discussed above, the most commonly used immunosuppressant is cyclosporine, which however has numerous side effects. Accordingly, in view of the relatively few choices for agents effective at immunosuppression with low toxicity profiles and manageable side effects, there exists a need in the art for identification of alternative immunosuppressive agents and for agents acting as complement to calcineurin inhibition.

The metastasis of cancer cells represents the primary source of clinical morbidity and mortality in the large majority of solid tumors. Metastasis of cancer cells may result from the entry of tumor cells into either lymphatic or blood vessels. Invasion of lymphatic vessels results in metastasis to regional draining lymph nodes. From the lymph nodes, melanoma cells for example tend to metastasize to the lung, liver, and brain. For several solid tumors, including melanoma, the absence or the presence of lymph nodes metastasis is the best predictor of patient survival. Presently, to our knowledge, no treatment is capable of preventing or significantly reducing metastasis. Hence, there is a need in the art for compounds having such anti-metastasis effect for a suitable treatment of cancer patients.

Septic shock is a major cause of death in intensive care units (about 150,000 estimated deaths annually in the United States of America, despite treatment with intravenous antibiotics and supportive care) for which very little effective treatment is available at present. Patients with severe sepsis often experience failures of various systems in the body, including the circulatory system, as well as kidney failure, bleeding and clotting. Lipopolysaccharide (hereinafter referred as LPS) is the primary mediator of Gramm-negative sepsis, the most common form of sepsis, by inducing the production of a whole array of macrophage-derived cytokines (such as TNF-α; interleukins such as IL-1, IL-6, IL-12; interferon-gamma (hereinafter referred IFN-γ), etc.). These cytokines may induce other cells (e.g. T cells, NK cells) to make cytokines as well (e.g. IFN-γ). In addition, other macrophage products (e.g. nitric oxide, hereinafter referred as NO) may also play a role in the pathogenesis of toxic shock. These substances (e.g. NO) may be induced directly due to microbial interactions or indirectly through the action of proinflammatory cytokines. LPS binds to a serum protein known as LPB and the LPS-LPB complex thus formed is recognized by the CD14 toll-like receptor 4 (hereinafter referred as Tlr 4) complex on mononuclear phagocytes. Tlr4 is a signal transducing unit, the activation of which results in the release of mediators such as TNF-α, IL-1α, IL-1β and IL-6. These cytokines are important for the pathogenesis of shock. Their administration produces the clinical symptoms of septic shock and their blockade partially protects against LPS-induced lethal shock.

Current therapeutic strategies for the treatment of septic shock are directed against LPS (e.g. antibodies against

LPS or LBP-34-23) or against the cytokines induced by LPS (e.g. TNF antibodies) or against the receptor for LPS (e.a. CD14). Unfortunately the initial clinical data of these approaches are very disappointing and illustrate the redundancy of receptors and mediators involved in the pathogenesis of toxic shock. For instance flagellin seems to be another toxin that plays a role in Gramm-negative Salmonella shock syndrome and that cannot be prevented or treated by therapeutic strategies directed specifically at LPS.

Clinical trials in humans with TNF-α blocking antibodies (such as the IL-1 receptor antagonist or PAF receptor antagonists) have been unsuccessful yet, as have been approaches to down regulate inflammation (e.g. using prednisolone) or to block endotoxins. These products must be administered very early after the onset of the disease, which is in most cases not possible.

The only drug currently approved by health authorities for the treatment of adult patients with the most serious forms of sepsis, including septic shock, is a genetically engineered version of a naturally occurring human protein, Activated Protein C, known as Xigris® or drotecogin-alpha which shows only moderate efficacy. Furthermore, because Activated Protein C interferes with blood clotting, the most serious side effect associated with Xigris® is bleeding, including bleeding that causes stroke. Thus Xigris® is contra-indicated for patients who have active internal bleeding, or who are more likely to bleed because of certain medical conditions including recent strokes, recent head or spinal surgery or severe head trauma. Beacause treatment with Xigris® comes with potentially serious risks, the benefits and risks of treatment with Xigris® must be carefully weighed for each individual patient.

Therefore there is a strong need in the art for new medications, either alone or in combination with the currently suggested treatments, for treating the most serious forms of life-threatening illnesses caused by severe infection, such as septic shock.

TNF-α is generally considered to be the key mediator in the mammalian response to bacterial infection. It is a strong pro-inflammatory agent that will affect the function of almost any organ system, either directly or by inducing the formation of other cytokines like IL-1 or prostaglandines. TNF-α is also a potent anti-tumor agent. If administered in small quantities to humans, it causes fever, headache, anorexia, myalgia, hypotension, capillary leak syndrome, increased rates of lipolysis and skeletal muscle protein degradation (including cachexia). Its use in cancer treatment is therefore very much limited by its severe side effects.

TNF-α, a pleiotropic cytokine produced mainly by activated macro-phages, exerts an *in vitro* cytotoxic action against transformed cells and *in vivo* anti-tumor activities in animal models. However, despite the fact that TNF-α is used in cancer patients especially to treat melanoma and sarcoma, the major problem hampering its use is toxicity. Indeed, TNF-α induces shock-like symptoms such as bowel swelling and damage, liver cell necrosis, enhanced release of inflammatory cytokines such as IL-1 or IL-6, and hypo-tension probably due to the release of inducers of vessels dilatation such nitric oxide and other proinflammatory cytokines. Cardiovascular toxicity is usually dose-limiting. Hypotension can be severe with systolic blood pressure below 60 mm Hg. Respiratory compromise is common after treatment with TNF-α and may require mechanical ventilation. Upper as well as lower digestive tract symptoms are also common in this type of treatment. Nausea and vomiting can be distressing and in some cases dose-limiting. Watery diarrhea is frequently observed. Neurological sequelae of treatment with TNF-α can also occur.

Hence, compounds that inhibit the toxic effects of TNF-α but that do not inhibit TNF-α anti-tumor effect are highly desirable for the treatment of cancer patients. Presently, several clinical trials involving TNF-α are being developed for the cancer of organs such as liver, lung, kidney and pancreas, which are based on a procedure including the steps of organ isolation, injection of TNF-α into the isolated organ, and reperfusion of the treated organ. However, even for isolated organ perfusion, some TNF-α usually escapes to the general blood circulation and leads to the mortality of about 10% of the patients thus treated. Many patients treated by this procedure also require intensive care unit rescue to cope with the toxic side-effects of such TNF-α treatment.

Combined treatment of TNF-α with alkylating drugs in an isolated organ perfusion model has received considerable attention. TNF-α is currently successfully used in isolated limb perfusion of human cancer patients and, in combination with melphalan and interferon-gamma, against melanoma, sarcomas and carcinomas.

The gastrointestinal mucosa is very sensitive to chemotherapeutic drugs. Mucositis caused by chemotherapy usually begins rapidly after initiation of the treatment with inflammation and ulceration of the gastrointestinal tract and leading to diarrhea. Severe, potentially life-threatening, diarrhea may require interruption of the chemotheraputic treatment and subsequent dose reduction of the therapeutic agent. The oral cavity is often the place of severe side effects from cancer therapy that adversely affects the quality of life of the patient and its ability to tolerate the therapy. These side effects can be caused by radiotherapy as well as chemotherapy. A relationship between both serum and mucosal levels of TNF-α and IL-1 correlates with nonhematologic toxicities, including mucositis.

Radiation injuries occurring e.g. after a single high-dose irradiation include apoptosis as well as radiation necrosis. Even normal tissues protected by shielding during irradiation may be considerably damaged. It was found in experimental animal models that the radiation injuries after a single high-dose irradiation typically used for the treatment of various malignant tumors consist of radiation necrosis and apoptosis, which were correlated with the expression of TNF-α and TGF-β1.

Irradiation may induce graft-versus-host disease (hereinafter referred as GVHD) in cancer patients. This disease may occur especially in patients receiving allogeneic bone marrow transplantation as a treatment for cancers such as leukemia or lymphoma and can lead to the death of about 25% of the relevant patients. Before bone marrow transplantation, leukaemia patients for example receive either total body or total lymphoid irradiation to suppress their immune system. However, such irradiation induces not only necrosis but also the release of proinflammatory cytokines mainly TNF-$\alpha$, IL-1 and IL-6 which in turn induce direct host tissues inflammation and activation of donor cells against host antigens leading to GVHD.

Cisplatin is an effective chemotherapeutic agent used in the treatment of a wide variety of both pediatric and adult malignancies, including testicular, germ cell, head and neck (cervical), bladder and lung cancer. Dose-dependent and cumulative nephrotoxicity is the major side effect of cisplatin, sometimes requiring a reduction in dose or discontinuation of the treatment. Other side effects of cisplatin include kidney damage, loss of fertility, harmful effect on a developing baby, temporary drop in bone marrow function causing drop in white blood cell count, anaemia, drop in platelets causing bleeding, loss of appetite, numbness or tingling in limbs, loss of taste, allergic reactions, and hearing disorders (difficulty in hearing some high-pitched sounds, experiencing ringing in the ears). Blurred vision may also be a side effect with high doses of cisplatin. It was shown that TNF-$\alpha$ is a key element in a network of proinflammatory chemokines and cytokines activated in the kidney by cisplatin. Blockade of TNF-$\alpha$ action would prevent the activation of this cytokine network and would provide protection against cisplatin nephrotoxicity. Hence, compounds that inhibit the toxic effects of cisplatin but that do not inhibit cisplatin anti-tumor effects are highly desirable for the treatment of cancer patients.

A surplus of TNF-$\alpha$ also causes a dramatic change of endothelial cells. In particular, TNF-$\alpha$ is an important mediator of skeletal muscle degeneration associated with cachexia, a debilitating syndrome characterized by extreme weight loss and whole-body wasting. Cachexia is usually a secondary condition whereby there is excessive tissue catabolism in combination with deficient anabolism. It is frequently seen in patients afflicted with chronic diseases such as cancer, cardiopulmonary diseases, aging, malabsortive disorders, excessive physical stress, easting disorders and acquired immmuno-deficiency syndrome (AIDS). Some authors consider that the elevated TNF-$\alpha$ values found in at least 50% of cancer patients in the active stage of the disease can result in cachexia. TNF-$\alpha$ levels in clinically healthy adults, as well as in adult cancer patients, are well documented, for instance by Nenova et al. in Archives of Hellenic Medicine (2000) 17:619-621. Serum TNF-$\alpha$ concentrations in healthy children as well as in children with malignancies are documented for instance by Saarinen et al. in Cancer Research (1990) 50:592-595. A very significant proportion of cancer mortalities result from cachexia rather than from tumor burden. Chronic wasting disease (cachexia) may result when excessive cellular damage results in the release of substances (TNF-$\alpha$, collagenase, hyaluronidase) that further catabolize the so-called healthy tissue resulting in an inability to assimilate nutrients required for anabolic restructuring of associated tissue.

Very few drugs have been suggested at present for the treatment of cachexia. Some high-dose progestins like megestrol acetate (an agent used for the treatment of metastatic breast cancer) and medroxyprogesterone acetate were shown in randomized clinical trials to provide a statistically significant advantage as regards improved appetite and body weight gain. Hence, compounds that stimulate appetite and body weight gain without inhibiting the anti-tumor effect of co-administered drugs are highly desirable for the treatment of cachexia. More specifically, there is a need in the art for treating cachexia by the administration of compounds that reduce TNF-$\alpha$ levels in the serum of humans.

TNF-$\alpha$ is also suspected to play a role, through a possible dual action in the hematopoietic environment, in the development of hematologic malignancies such as idiopathic myelodysplastic syndromes occurring most often in elderly people but also occasionally in children, these syndromes being currently regarded as the early phase of acute leukemia.

There is a strong need in the art to improve, or to provide alternatives to, the existing prophylactic or therapeutic solutions to all the aforesaid diseases. Meeting one or more of these various needs in the art constitutes the main goal of the present invention.

## SUMMARY OF THE INVENTION

The present invention is based on the unexpected finding that certain combinations of three specific classes of substituents on positions 2, 4 and 6 of the pyrido(2,3-d)pyrimidine ring (using the standard atom numbering for this moiety), said combinations not being suggested by the prior art, are able to meet one or more of the medical needs recited herein above and to show unexpected biological properties. Thus, an important feature of the present invention is a substitution pattern of the pyrido(2,3-d)pyrimidine ring which comprises, or alternatively which consists of, any of the following combinations of three substituents on positions 2, 4 and 6 of said ring. Because a 4-amino substitution of the pyrido(2,3-d)pyrimidine ring has been found unsuitable for obtaining certain desirable biological properties, another important feature of the present invention is a substitution pattern of the pyrido(2,3-d)pyrimidine ring wherein the substituent on position 4 of said ring is not amino ($NH_2$).

Based on this finding the present invention relates, in a broad expession of its first aspect, to a class of novel trisubstituted pyrido(2,3-d)pyrimidine derivatives having the structural formula (I):

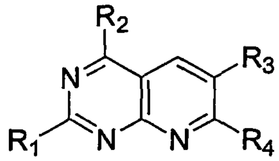

wherein:

- $R_1$ is amino ;
- $R_2$ is selected from the group consisting of hydroxy, halogen, mono-$C_{1-7}$ alkylamino; mono- arylamino; mono-aryl$C_{1-7}$alkylamino; morpholinyl; N-piperidinyl; triazolyl; heterocyclic-substituted amino; $C_{1-7}$alkoxy; oxyheterocyclic; piperazinyl or homopiperazinyl, wherein said piperazinyl is optionally N-substituted with acyl $C_{1-7}$ alkyl or arylalkyl;
- $R_3$ is selected from the group consisting of aryl and heteroaryl groups optionally substituted with one or more substituents selected from the group consisting of halogen, halo $C_{1-7}$ alkyl, $C_{1-7}$ alkoxy and alkylamino; and
- $R_4$ is hydrogen,

or a pharmaceutical acceptable addition salt thereof, or a dihydro-derivative or a tetrahydro-derivative thereof, or a stereoisomer thereof, or a *N*-oxide thereof, or a solvate thereof.

In a second aspect, the present invention relates to certain groups of tri-substituted pyrido(2,3-d)pyrimidines which are useful as intermediates for making some of the biologically active trisubstituted pyrido(2,3-d)pyrimidine derivatives having the structural formula (I). As shown in appended figure 1, such groups include, but are not limited to:

- a group of 2-amino-4-hydroxy-6-$R_3$-substituted pyrido(2,3-d)pyrimidines wherein $R_3$ is as defined in formula (I), and tautomers thereof;

wherein R3 is as defined in formula (I).

In a third aspect, the present invention relates to the unexpected finding that at least one desirable biological property such as, but not limited to, the ability to decrease the proliferation of lymphocytes, or to decrease T-cell activation, or to decrease B-cell or monocytes or macrophages activation, or to inhibit the release of certain cytokines, or in inhibiting human TNF-$\alpha$ production, is a feature which is present in the class of novel trisubstituted pyrido(2,3-d)pyrimidine compounds having the structural formula (I) such as, but not limited to, those compounds wherein:

- $R_1$ is amino ;
- $R_2$ is selected from the group consisting of mono-$C_{1-7}$ alkylamino; mono-arylamino; mono-aryl$C_{1-7}$alkylamino; morpholinyl; N-piperidinyl; triazolyl; heterocyclic-substituted amino; $C_{1-7}$ alkoxy; oxyheterocyclic; piperazinyl or homopiperazinyl, wherein said piperazinyl is optionally N-substituted with acyl, $C_{1-7}$ alkyl or arylalkyl;
- $R_3$ is selected from aryl and heteroaryl groups optionally substituted with one or more substituents selected from the group consisting of halogen, halo $C_{1-7}$ alkyl, $C_{1-7}$ alkoxy and alkylamino; and
- $R_4$ is hydrogen,

including pharmaceutical acceptable addition salts thereof, dihydro- or tetrahydro-derivatives thereof, stereoisomers thereof, N-oxides thereof, and/or solvates thereof.

As a consequence, the present invention also relates to pharmaceutical compositions comprising as an active principle at least one trisubstituted pyrido(2,3-d)pyrimidine compound having the structural formula (I) with the above meanings of $R_1$, $R_2$, $R_3$ and $R_4$, and/or a pharmaceutically acceptable addition salt thereof, and/ or a dihydro-derivative or a tetrahydro-derivative thereof, and/or a stereoisomer thereof, and/or a N-oxide thereof, and/or a solvate thereof.

In particular, these trisubstituted pyrido(2,3-d)pyrimidine compounds having the structural formula (I) with the above meanings of $R_1$, $R_2$, $R_3$ and $R_4$ are highly active immunosuppressive agents, or antineoplastic agents which, together with one or more pharmaceutically acceptable carriers, may be formulated into pharmaceutical compositions suitable for the prevention or treatment of pathologic conditions such as, but not limited to, immune and autoimmune disorders, organ and cells transplant rejections, cell proliferative disorders, cardiovascular disorders and disorders of the central nervous system. Trisubstituted pyrido(2,3-d)pyrimidine compounds having the structural formula (I) with the above meanings of $R_{1,}$ $R_2$, $R_3$ and $R_4$ are also useful as active ingredients for the manufacture of medicaments for the prevention or treatment of a TNF-$\alpha$-related disorder in a mammal, such as for instance:

- septic or endotoxic shock,

- TNF-α- mediated diseases,
- pathologies and conditions associated with and/or induced by abnormal levels of TNF-α occurring in a systemic, localized or particular tissue type or location in the body of the mammal,
- toxic effects of TNF-α and/or anti-cancer chemotherapeutic agents,
- injuries after irradiation of a tissue of the mammal by radio-elements, and
- cachexia.

In a further aspect, the present invention also relates to combined preparations containing at least one trisubstituted pyrido(2,3-d)pyrimidine compound having the structural formula (I) with the above meanings of $R_1$, $R_2$, $R_3$ and $R_4$ together with one or more drugs such as, but not limited to, immunosuppressant and/or immunomodulator drugs, antineoplastic drugs, anti-histamines or inhibitors of agents causative of allergic conditions. In a further aspect, the present invention relates to methods for the prevention or treatment of one or more of the above-mentioned disorders or pathologic conditions, said methods being performed by administering to a patient (e.g. a human being) in need thereof an effective amount of a trisubstituted pyrido(2,3-d)pyrimidine compound having the structural formula (I) with the above meanings of $R_1$, $R_2$, $R_3$ and $R_4$, optionally in the form of a pharmaceutical composition with pharmaceutically acceptable excipients and/or a combined preparation together with an effective amount of another suitable drug.

In yet another aspect, the present invention relates to various processes and methods for making the novel trisubstituted pyrido(2,3-d)pyrimidine compounds defined in the structural formula (I) with the above meanings of $R_1$, $R_2$, $R_3$ and $R_4$ as well as their pharmaceutically acceptable salts, N-oxides, solvates and stereoisomers, e.g. via one or more tri-substituted pyrido(2,3-d)pyrimidine intermediates such as specified herein before.

BRIEF DESCRIPTION OF THE DRAWINGS

Figure 1 schematically shows methods for making 2,4,6-trisubstituted pyrido(2,3-d)pyrimidine derivatives having the formula (I).

Figure 2 schematically shows methods for making 2,4,6-trisubstituted pyrido(2,3-d)pyrimidine derivatives having the formula (I) wherein the substituent in position 4 is a piperazin-1-yl group being functionalized on its second nitrogen atom.

DEFINITIONS

Unless otherwise stated herein, the term " trisubstituted " means that at least the three carbon atoms being in positions 2, 4 and 6 of the pyrido(2,3-d)pyrimidine moiety (according to standard atom numbering for the pyrido(2,3-d) pyrimidine moiety) are substituted with an atom or group of atoms other than hydrogen.

As used herein with respect to a substituting radical, and unless otherwise stated, the term " $C_{1-7}$ alkyl " means straight and branched chain saturated acyclic hydrocarbon monovalent radicals having from 1 to 7 carbon atoms such as, for example, methyl, ethyl, propyl, n-butyl, 1-methylethyl (isopropyl), 2-methylpropyl (isobutyl), 1,1-dimethylethyl (ter-butyl), 2-methylbutyl, n-pentyl, dimethylpropyl, n-hexyl, 2-methylpentyl, 3-methylpentyl, n-heptyl and the like; the term " $C_{1-4}$ alkyl " refers to a group of such radicals having from 1 to 4 carbon atoms, and so on.

As used herein with respect to a substituting radical, and unless otherwise stated, the term " acyl " broadly refers to a carbonyl (oxo) group being adjacent to a $C_{1-7}$ alkyl radical, an aryl radical, an arylalkyl radical or a heterocyclic radical, all of them being such as herein defined, including sub-groups thereof; representative examples of acyl include, but are not limited to, acetyl, pivaloyl, benzoyl, naphthoyl and the like.

As used herein with respect to a substituting radical, and unless otherwise stated, the term " aryl " designates any mono- or polycyclic aromatic monovalent hydrocarbon radical having from 6 up to 30 carbon atoms such as, but not limited to, phenyl, naphthyl, anthracenyl, phenanthracyl, fluoranthenyl, chrysenyl, pyrenyl, biphenylyl, terphenyl, picenyl, indenyl, biphenyl, indacenyl, benzocyclobutenyl, benzocyclooctenyl and the like, including fused benzo-$C_{4-8}$ cycloalkyl radicals such as, for instance, indanyl, tetrahydronaphtyl, fluorenyl and the like, all of the said radicals being optionally substituted with one or more substituents independently selected from the group consisting of halogen, halo $C_{1-7}$ alkyl and $C_{1-7}$ alkoxy (all of them being such as herein defined, including sub-groups thereof), such as for instance 4-fluorophenyl, 4-chlorophenyl, 3,4-dichlorophenyl, 2,6-dichlorophenyl, 2-fluorophenyl, 3-chlorophenyl, 3,5-dichlorophenyl, trifluoromethylphenyl and 3,4-dimethoxyphenyl.

As used herein with respect to a substituting radical, and unless otherwise stated, the term " heterocyclic " means a mono- or polycyclic, saturated or mono-unsaturated or polyunsaturated monovalent hydrocarbon radical having from 2 up to 15 carbon atoms and including one or more heteroatoms in one or more heterocyclic rings, each of said rings having from 3 to 10 atoms (and optionally further including one or more heteroatoms attached to one or more

carbon atoms of said ring, for instance in the form of a carbonyl, and/or to one or more heteroatoms of said ring, for instance in the form of a sulfone, sulfoxide or N-oxide), each of said heteroatoms being independently selected from the group consisting of nitrogen, oxygen and sulfur, also including radicals wherein a heterocyclic ring is fused to one or more aromatic hydrocarbon rings for instance in the form of benzo-fused, dibenzo-fused and naphto-fused heterocyclic radicals; within this definition are included heterocyclic radicals such as, but not limited to, thienyl, piperidinyl, morpholinyl, thiomorpholinyl, triazolyl, piperazinyl, homopiperazinyl, homopiperidinyl, and the like, wherein each carbon atom of said heterocyclic ring may furthermore be independently substituted; depending upon the number of unsaturations in the 3 to 10 atoms ring, heterocyclic radicals may be sub-divided into heteroaromatic (or " heteroaryl ") radicals and non-aromatic heterocyclic radicals according to standard knowledge in the art; when a heteroatom of said heterocyclic radical is nitrogen, the latter may be substituted with a substituent selected from the group consisting of $C_{1-7}$ alkyl, aryl or arylalkyl and alkylaryl (all of them being such as herein defined, including sub-groups thereof).

As used herein with respect to a substituting radical, and unless otherwise stated, the terms " $C_{1-7}$ alkoxy ", " aryloxy ", " arylalkyloxy " and " oxyheterocyclic " refer to substituents wherein a carbon atom of a $C_{1-7}$ alkyl, respectively an aryl, arylalkyl or heterocyclic radical (each of them such as defined herein, including sub-groups thereof), is attached to an oxygen atom through a single bond such as, but not limited to, methoxy, ethoxy, propoxy, n-butoxy, pentoxy, isopropoxy, sec-butoxy, tert-butoxy, isopentoxy, phenoxy, benzyloxy, piperidinoxy and the like.

As used herein with respect to a substituting atom, and unless otherwise stated, the term halogen means any atom selected from the group consisting of fluorine, chlorine, bromine and iodine.

As used herein with respect to a substituting radical, and unless otherwise stated, the term " halo $C_{1-7}$ alkyl " means a $C_1$-$_7$ alkyl radical (such as above defined, including sub-groups thereof) in which one or more hydrogen atoms are independently replaced by a corresponding number of halogen atoms (preferably fluorine, chlorine or bromine) such as, but not limited to, fluoromethyl, difluoromethyl, trifluoromethyl, chloromethyl and the like.

As used herein with respect to a substituting radical, and unless otherwise stated, the term " arylalkyl " refers to an aliphatic saturated hydrocarbon monovalent radical (preferably a $C_{1-7}$ alkyl radical such as defined above, including sub-groups thereof) onto which an aryl radical (such as defined above, including sub-groups thereof) is already bonded such as, but not limited to, benzyl, 4-chlorobenzyl, 4-fluorobenzyl, 2-fluorobenzyl, 3,4-dichlorobenzyl, 2,6-dichlorobenzyl, and phenylethyl.

As used herein with respect to a substituting radical, and unless otherwise stated, the terms " mono-alkylamino ", " mono-arylamino ", " mono-arylalkylamino " mean that one $C_{1-7}$ alkyl, aryl, arylalkyl or heterocyclic (provided in the latter case that the nitrogen atom of the the amino group is attached to a carbon atom of the heterocyclic ring) radical (each of them as defined herein, respectively) is/are attached to a nitrogen atom through a single bond such as, but not limited to, anilino, benzylamino, methylamino, ethylamino, isopropylamino, n-butylamino, ter-butylamino and bromoanilino.

As used herein and unless otherwise stated, the term " stereoisomer " refers to all possible different isomeric as well as conformational forms which the compounds of formula (I) may possess, in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

As used herein and unless otherwise stated, the term " enantiomer " means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

As used herein and unless otherwise stated, the term " solvate " includes any combination which may be formed by a trisubstituted pyrido(2,3-d)pyrimidine derivative of this invention with a suitable inorganic solvent (e.g. hydrates formed from water) or a suitable organic solvent such as, but not limited to, alcohols, ketones, esters, ethers, nitriles and the like.

As used herein and unless otherwise stated, the terms " dihydro-derivative " and " tetrahydro-derivative " refers to the hydrogenation products of the trisubstituted pyrido(2,3-d)pyrimidine derivatives having the structural formula (I), i.e. derivatives wherein either two more hydrogen atoms are present in positions 5 and 6, or 7 and 8, of the pyrido(2,3-d)pyrimidine ring, or respectively wherein four more hydrogen atoms are present in positions 5, 6, 7 and 8 of the said ring; such hydrogenated pyrido(2,3-d)pyrimidine derivatives are easily accessible from the derivatives using hydrogenation methods well known in the art.

## DETAILED DESCRIPTION OF THE INVENTION

As explained in the above summary, the present invention relates, in a first aspect, to a broad class of novel compounds having the structural formula (I) wherein each of $R_1$, $R_2$ and $R_3$ is independently selected as specified herein above, and wherein $R_4$ is hydrogen.

Within the above defined class of trisubstituted pyrido(2,3-d)pyrimidine compounds of this invention, a sub-group comprises novel compounds having the structural formula (I) wherein $R_1$, $R_3$ and $R_4$ are as specified herein above and wherein $R_2$ is halogen or triazolyl, which are mainly useful as intermediates for making biologically-active trisubstituted pyrido(2,3-d)pyrimidine compounds having the structural formula (I) wherein $R_1$, $R_3$ and $R_4$ are as specified herein above, and wherein $R_2$ is as specified herein above, except for halogen or triazolyl.

Such sub-group of compounds of this invention are illustrated in the appended figures 1 and 2 and in the following detailed description namely with, but not limited to, $R_3$ being bromo and/or $R_2$ (when designated as L) being chloro or triazolyl.

An embodiment of the broad class of trisubstituted pyrido(2,3-d)pyrimidine compounds of this invention is a sub-group of compounds having the structural formula (I) wherein $R_1$ is amino and wherein $R_2$, $R_3$ and $R_4$ are as specified in the broadest expression herein above.

Another embodiment of the broad class of trisubstituted pyrido(2,3-d)pyrimidine compounds of this invention is a sub-group of compounds having the structural formula (I) wherein $R_1$ is amino, wherein $R_2$ and $R_4$ are as specified in the broadest expression herein above and wherein $R_3$ is a monosubstituted or polysubstituted phenyl group (the one or more substituent(s) of said phenyl group being as defined herein before).

Another embodiment of the broad class of trisubstituted pyrido(2,3-d)pyrimidine compounds of this invention is a sub-group of compounds having the structural formula (I) wherein $R_1$ is amino, wherein $R_2$ is morpholinyl; N-piperidinyl; triazolyl; piperazinyl or homopiperazinyl, wherein said piperazinyl is optionally N-substituted with acyl or arylalkyl (such as, but not limited to, benzylpiperazinyl), and wherein $R_3$ is a monosubstituted or polysubstituted phenyl group (the one or more substituent(s) of said phenyl group being as defined herein before).

Another embodiment of the broad class of trisubstituted pyrido(2,3-d)pyrimidine compounds of this invention is a sub-group of compounds having the structural formula (I) wherein $R_1$ is amino, wherein $R_2$ is oxyheterocyclic (such as, but not limited to, tetrahydro-pyranyloxy or methylpiperidinoxy), and wherein $R_3$ is a monosubstituted or polysubstituted phenyl group (the one or more substituent(s) of said phenyl group being as defined herein before).

Another embodiment of the broad class of trisubstituted pyrido(2,3-d)pyrimidine compounds of this invention is a sub-group of compounds having the structural formula (I) wherein $R_1$ is amino, wherein $R_2$ is $C_{1-7}$ alkoxy (preferably $C_{1-4}$ alkoxy such as, but not limited to, methoxy, propoxy or butoxy), and wherein $R_3$ is a monosubstituted or polysubstituted phenyl group (the one or more substituent(s) of said phenyl group being as defined herein before).

Another embodiment of the broad class of trisubstituted pyrido(2,3-d)pyrimidine compounds of this invention is a sub-group of compounds having the structural formula (I) wherein $R_1$ is amino, wherein $R_2$ is mono-$C_{1-7}$ alkylamino (preferably monopropylamino), mono-arylamino (preferably anilino or bromoanilino) or mono-aryl$C_{1-7}$alkylamino (preferably benzylamino or phenylethylamino), and wherein $R_3$ is a monosubstituted or polysubstituted phenyl group (the one or more substituent(s) of said phenyl group being as defined herein before).

Another embodiment of the broad class of trisubstituted pyrido(2,3-d)pyrimidine compounds of this invention is a sub-group of compounds having the structural formula (I) wherein $R_1$ is amino, wherein $R_2$ and $R_4$ are as specified in the broadest expression herein above and wherein $R_3$ is a phenyl group having no more than two substituents as defined hereinabove, preferably wherein one substituent is in a para position on said phenyl ring.

In the first aspect of the present invention, the novel 2,4,6-trisubstituted pyrido(2,3-d)pyrimidine derivatives are as defined in the structural formula (I), wherein each of the substituents $R_1$, $R_2$ and $R_3$ may independently correspond to any of the definitions given above, in particular with any of the individual meanings (such as illustrated above) of generic terms used for substituting atoms or radicals such as, but not limited to, " $C_{1-7}$ alkyl ", " aryl ", " heterocyclic ", " halogen ", "arylalkyl ", "alkylamino", " arylamino ", " arylalkylamino ", " oxyheterocyclic ", " heterocyclic-substituted amino ", " $C_{1-7}$ alkoxy ", " halo $C_{1-7}$ alkyl" and the like.

In the second aspect of the present invention, the novel 2,4,6-trisubstituted pyrido(2,3-d)pyrimidine intermediates are as specified herein, especially as shown in the figures, wherein each of the substituents $R_1$, $R_2$, $R_3$ and/or L may independently correspond to any of the definitions given with respect to the general formula (I) or in the following process description, in particular with any of the individual meanings (such as illustrated above) of generic terms used for substituting atoms or radicals such as, but not limited to, " $C_{1-7}$ alkyl ", " aryl ", " heterocyclic ", " oxyheterocyclic ", " halogen ", " arylalkyl ", " alkylamino ", " $C_{1-7}$ alkoxy ", " halo $C_{1-7}$ alkyl " and the like.

Within the class of trisubstituted compounds having the general formula (I), an important group is one wherein $R_2$ is a piperazinyl group optionally N-substituted with a substituent $R_5$ such as defined herein above. Said piperazinyl group may be further substituted, at one or more carbon atoms, by a number n of substituents $R_0$ wherein n is an integer from 0 to 4 and wherein, when n is at least 2, each $R_0$ may be defined independently from the others. The presence of one or more such substituents $R_0$ at one or more carbon atoms may be a suitable way for introducing chirality into the trisubstituted pyrido(2,3-d)pyrimidine derivatives having the general formula (I) as well as into the corresponding intermediates for making them. In practice, the choice of such substituents $R_0$ may be restricted by the commercial availability of the substituted piperazine. More preferably $R_2$ is a piperazin-1-yl group, n is 0, 1 or 2, and a representative example of the substituent $R_0$ is methyl or phenyl such as for instance in 2-methylpiperazin-1-yl, 2-phenylpiperazin-1-yl and 2,5-dimethyl-piperazin-1-yl. Within this group of compounds, a more specific embodiment of the invention is one wherein

one of the two nitrogen atoms of the piperazinyl group bears a substituent $R_5$ which has a carbonyl (oxo) function preferably immediately adjacent to the said nitrogen atom. In other words, this specific embodiment means that when $R_5$ is selected from, respectively, acyl, amide or carboxylate, then $R_5$ together with the nitrogen atom to which it is attached forms, respectively, an amide, urea or carbamato group.

Especially useful species of trisubstituted pyrido(2,3-d)pyrimidine derivatives having the general formula (I) are those wherein the substituent $R_2$ is a piperazin-1-yl group, said group being substituted in the 4 position with a substituent $R_5$, wherein $R_5$ is selected from the group consisting of:

- $COR_8$ wherein $R_8$ is selected from hydrogen; $C_{1-7}$ alkyl; aryl optionally substituted with one or more substituents (as defined hereinabove); aryloxy; and arylamino; representative but non limiting examples of $R_8$ include benzyloxy, phenoxy, and the like; a representative but non limiting example of $COR_8$ includes methylphenylcarbamoyl; and
- $R_{11}$, wherein $R_{11}$ is arylalkyl such as but not limited to benzyl.

The present invention further provides various processes and methods for making the novel trisubstituted pyrido (2,3-d)pyrimidine derivatives having the structural formula (I). As a general rule, the preparation of these compounds is based on the principle that, starting from a suitable pyrido(2,3-d)pyrimidine precursor (usually a 2-$R_1$-substituted-6-amino-pyrimidin-4-one), each of the other substituents $R_2$ and $R_3$ may be introduced separately without adversely influencing the presence of one or more substituents already present at other positions on the pyrido(2,3-d)pyrimidine moiety or the capacity to introduce further substituents later on during the synthetic procedure.

Methods of manufacture have been developed by the present inventors which may be used alternatively to, or may be combined at will with, the methods of synthesis already known in the art for 2,4,6-trisubstituted pyrido(2,3-d) pyrimidine derivatives, depending upon the targeted final compound. For instance, the synthesis of mono-N-oxides of the pyrido(2,3-d)pyrimidine derivatives of this invention can easily be achieved by treating said derivatives with one or more oxidizing agents such as, but not limited to, hydrogen peroxide (e.g. in the presence of acetic acid) or a peracid such as chloroperbenzoic acid, under conditions well known to the person skilled in the art.

Several methods for making the trisubstituted pyrido(2,3-d)pyrimidine derivatives of the present invention will now be explained in more details by reference to the appended figure 1 wherein, unless otherwise stated hereinafter, each of the substituting groups or atoms $R_1$, $R_2$ and $R_3$ is independently as defined with respect to structural formula (I) of the summary of the invention and, more specifically, wherein each of $R_1$, $R_2$ and $R_3$ may correspond to any of the individual meanings disclosed above.

In the description of the reaction steps involved in figure 1, reference is made by way of example to the use of certain catalysts and/or certain types of solvents. It should be understood that the type of catalyst is usually not critical to the performance of the corresponding reaction step (except maybe in terms of the yield achieved), and that each catalyst mentioned should be used in a catalytic amount well known to the skilled person with respect to the type of reaction involved. Solvents that may be used in the following reaction steps include various kinds of organic solvents such as, but not limited to, protic solvents, polar aprotic solvents and non-polar solvents as well as aqueous solvents which are inert under the relevant reaction conditions. More specific examples of suitable solvents include, but are not limited to, aromatic hydrocarbons, chlorinated hydrocarbons, ethers, aliphatic hydrocarbons, alcohols, esters, ketones, amides, water or mixtures thereof, as well as supercritical solvents such as carbon dioxide (while performing the reaction under supercritical conditions). The suitable reaction temperature and pressure conditions, as well as the choice of the most appropriate solvent, applicable to each kind of reaction step will not be detailed herein but do not depart from the relevant conditions and solvents already known to the skilled person with respect to the type of reaction involved and the type of solvent used (in particular its boiling point). Also, although the various reaction steps desirable for making the compounds of the invention in good yield and purity are shown and described herein in a certain order, the skilled person may also be able to arrive at the same or a similar result while changing the order of the reaction steps with respect to the illustrative scheme presented herein.

Figure 1 schematically shows a method for making 2,4,6-trisubstituted pyrido(2,3-d)pyrimidine derivatives having the formula (I), as well as intermediates therefor wherein the substituent in position 2 may be a N-protected amino such as acylamino (e.g. acetamido or pivalamido), and/or wherein the substituent in position 4 may be hydroxy, halogen (in particular chloro) or triazolyl. In a first step (a), a 2-$R_1$-substituted-6-amino-pyrimidin-4-(3*H*)one is condensed with bromomalonaldehyde or chloromalonaldehyde, yielding the desired 2-$R_1$-substituted-4-oxo-6-bromopyrido [2,3-d]pyrimidine (shown in figure 1) or 2-$R_1$-substituted-4-oxo-6-chloro-pyrido[2,3-d]pyrimidine derivative (not shown).

The bromine or chlorine atom at position 6 of the pyrido[2,3-d]pyrimidine scaffold is a substituent suitable for further derivatisation in a palladium catalysed reaction such as, but not limited to, a Suzuki reaction. For instance, this substituting position of the reaction product of step (a) can be used efficiently in the following step (b) for reacting with an aryl boronic acid including the radical $R_3$, the aryl group of said aryl boronic acid being optionally substituted with one or more substituents (such as mentioned in the above definitions), thus leading to the formation of a 2-$R_1$-substituted-4-oxo-6-$R_3$-substituted-pyrido[2,3-d]pyrimidine derivative in good yield. Alternatively, said Suzuki reaction may be performed, as shown in figure 1, after introduction of the substitutent $R_2$ in step (d). Activation of the tautomeric hydroxyl group at position 4 of the pyrido[2,3-d]pyrimidine scaffold for the subsequent nucleophilic displacement reaction occurs in the following step (c) by preparing the corresponding 2-$R_1$-substituted-4-(1,2,4-triazolyl)-6-$R_3$-substituted-pyrido[2,3-d]pyrimidine or 2-$R_1$-substituted-4-(1,2,4-triazolyl)-6-bromo-pyrido[2,3-d]pyrimidine derivative (as illustrated in the following examples), e.g. using $POCl_3$ or 4-chlorophenyl phosphorodichloridate and 1,2,4-triazole in pyridine as a solvent. Alternatively, the said tautomeric hydroxyl group can also be activated by introduction of a chlorine atom, for example by treatment with thionyl chloride or $POCl_3$, then resulting in a 2-$R_1$-substituted-4-chloro-6-bromo-pyrido[2,3-d]pyrimidine or a 2-$R_1$-substituted-4-chloro-6-$R_3$-substituted-pyrido[2,3-d]pyrimidine derivative. The triazolyl group or the chlorine atom of both alternative embodiments are indicated by L in the appended figure 1.

When $R_1$ is an amino group, protection of $R_1$ may be desirable, recommended or even necessary before carrying out the reaction of step (c). Such an amino group can be effectively protected (while using protecting methods well known in the art) for instance by an acyl group (e.g. pivaloyl or acetyl), for instance by reaction with pivaloyl anhydride or acetic anhydride in pyridine as a solvent. This protecting step results into the introduction, at position 2 of the pyrido [2,3-d]pyrimidine scaffold, of a N-protected amino group such as, but not limited to, acetamido or pivalamido, which can be readily converted, e.g. hydrolysed, back to an amino group in a next step of the process when needed.

Nucleophilic substitution of the triazolyl group or chlorine atom is performed in following reaction step (d) by reacting the 2-$R_1$-substituted-4-(1,2,4triazolyl)-pyrido[2,3-d]pyrimidine or 2-$R_1$-substituted-4-chloro-pyrido[2,3-d]pyrimidine derivative with an appropriate nucleophile having the general formula $R_2Y$ wherein $R_2$ is as defined in the general formula (I) and wherein Y is hydrogen or an alcali metal. A suitable nucleophile includes for example an alkylamine, an arylamine, an arylalkylamine, morpholine, piperazine or a N-substituted piperazine (such as detailed herein), homopiperazine, piperidine or a substituted piperidine, sodium alkoxide, sodium aryloxide, sodium arylalkyloxide, and the like, and reaction step (d) is preferably performed at moderate temperature (usually about room temperature) in a polar aprotic solvent such as, but not limited to, 1,4-dioxane. Representative but non limiting examples of commercially available N-substituted piperazines that can suitably be used in step (d) of this method include benzylpiperazine and the like. In a final step (not shown in figure 1), any amino protecting group which may have been introduced previously is easily cleaved off by using standard cleavage conditions such as acidic or basic hydrolysis.

Thus, a method for making a trisubstituted pyrido(2,3-d)pyrimidine derivative or intermediate of this invention includes a critical step (b) of reacting a 2-$R_1$-substituted-6-bromo-pyrido[2,3-d]pyrimidine or a 2-$R_1$-substituted-6-chloro-pyrido[2,3-d]pyrimidine, wherein $R_1$ is as defined in the structural formula (I), with a (hetero)aryl boronic acid including a suitable aryl or heteroaryl radical $R_3$ as defined in the structural formula (I). The pyrido[2,3-d]pyrimidine starting material for this critical step (b) :

- either is already known in the art, e.g. from Taylor et al. in Synth. Commun. (1988) 18 :1187-1191 when $R_1$ is amino and an oxo group (tautomer to a hydroxyl group) is present in position 4 of the pyrido[2,3-d]pyrimidine scaffold, or
- may be prepared from the corresponding 2-$R_1$-substituted-6-amino-pyrimidin-4-(3H)one by analogy with the procedures described in said prior art reference when an oxo group (tautomer to a hydroxyl group) is present in position 4 of the pyrido[2,3-d]pyrimidine scaffold but $R_1$ is different from amino, or
- may be prepared from the above compounds after introduction of a substituent $R_2$ in position 4 of the pyrido[2,3-d] pyrimidine scaffold.

Figure 2 schematically shows methods for making 2,4,6-trisubstituted pyrido(2,3-d)pyrimidine derivatives having the formula (I) wherein the substituent in position 4 is a piperazin-1-yl group being functionalized on its second nitrogen atom. In deed, when the nucleophile used in step (d) of figure 1 has a second nucleophilic nitrogen atom (e.g. piperazine or homopiperazine), this second nitrogen atom can easily be acylated by treatment with an appropriate carboxylic acid chloride in an aprotic solvent such as, but not limited to, dimethylformamide, dichloromethane or pyridine, and, if necessary, in the presence of an effective amount of a base such as a tertiary amine (e.g. triethylamine). Alternatively, the free amino group of the piperazin-1-yl moiety may be transformed into a urea by reaction with an appropriate isocyanate in an aprotic solvent such as dimethylformamide or dichloromethane, or may be transformed into a carbamate by reaction with an appropriate chloroformate in an aprotic solvent such as, but not limited to, dimethylformamide, dichloromethane or pyridine and, if necessary, in the presence of an effective amount of a base such as a tertiary amine (e.g. triethylamine).

In another particular embodiment, the invention relates to a group of trisubstituted pyrido(2,3-d)pyrimidine derivatives having the above formula (I) and being in the form of a pharmaceutically acceptable salt. The latter includes any therapeutically active non-toxic addition salt which compounds having the formula (I) are able to form with a salt-forming agent. Such addition salts may conveniently be obtained by treating the trisubstituted pyrido(2,3-d)pyrimidine derivatives of the invention with an appropriate salt-forming acid or base. For instance, trisubstituted pyrido(2,3-d) pyrimidine derivatives having basic properties may be converted into the corresponding therapeutically active, non-toxic acid addition salt form by treating the free base form with a suitable amount of an appropiate acid following conventional

procedures. Examples of such appropriate salt-forming acids include, for instance, inorganic acids resulting in forming salts such as, but not limited to, hydrohalides (e.g. hydrochloride and hydrobromide), sulfate, nitrate, phosphate, diphosphate, carbonate, bicarbonate, and the like; and organic monocarboxylic or dicarboxylic acids resulting in forming salts such as, for example, acetate, propanoate, hydroxyacetate, 2-hydroxypropanoate, 2-oxopropanoate, lactate, pyruvate, oxalate, malonate, succinate, maleate, fumarate, malate, tartrate, citrate, methanesulfonate, ethanesulfonate, benzoate, 2-hydroxybenzoate, 4-amino-2-hydroxybenzoate, benzene-sulfonate, p-toluenesulfonate, salicylate, p-aminosalicylate, pamoate, bitartrate, camphorsulfonate, edetate, 1,2-ethanedisulfonate, fumarate, glucoheptonate, gluconate, glutamate, hexylresorcinate, hydroxynaphtoate, hydroxyethanesulfonate, mandelate, methylsulfate, pantothenate, stearate, as well as salts derived from ethanedioic, propanedioic, butanedioic, (Z)-2-butenedioic, (E)2-butenedioic, 2-hydroxybutanedioic, 2,3-dihydroxybutane-dioic, 2-hydroxy-1,2,3-propanetricarboxylic and cyclohexanesulfamic acids and the like.

Trisubstituted pyrido(2,3-d)pyrimidine derivatives having the general formula (I) having acidic properties may be converted in a similar manner into the corresponding therapeutically active, non-toxic base addition salt form. Examples of appropriate salt-forming bases include, for instance, inorganic bases like metallic hydroxides such as but not limited to those of alkali and alkaline-earth metals like calcium, lithium, magnesium, potassium and sodium, or zinc, resulting in the corresponding metal salt; organic bases such as but not limited to ammonia, alkylamines, benzathine, hydrabamine, arginine, lysine, N,N'-dibenzylethylenediamine, chloroprocaine, choline, diethanolamine, ethylenediamine, N-methylglucamine, procaine and the like.

Reaction conditions for treating the trisubstituted pyrido(2,3-d)pyrimidine derivatives having the general formula (I) of this invention with an appropriate salt-forming acid or base are similar to standard conditions involving the same acid or base but different organic compounds with basic or acidic properties, respectively. Preferably, in view of its use in a pharmaceutical composition or in the manufacture of medicament for treating specific diseases, the pharmaceutically acceptable salt will be designed, i.e. the salt-forming acid or base will be selected, so as to impart a greater water-solubility, a lower toxicity, a greater stability, a specific crystalline form and/or a better controlled dissolution rate to the trisubstituted pyrido(2,3-d)pyrimidine derivative of this invention.

The present invention further provides the use of a trisubstituted pyrido(2,3-d)pyrimidine derivative represented by the general formula (I), or a pharmaceutically acceptable salt thereof, or a dihydro-derivative or a tetrahydro-derivative thereof, or a stereoisomer thereof, or a *N*-oxide thereof, or a solvate thereof, as a biologically-active ingredient, i.e. active principle, especially as a medicinal agent or a diagnostic agent or for the manufacture of a medicament or a diagnostic kit. In particular the said medicament may be for the prevention or treatment, in a mammal such as a human being, of a pathologic condition selected from the group consisting of:

- immune disorders, in particular organ and cells transplant rejections, and autoimmune disorders,
- cardiovascular disorders,
- disorders of the central nervous system,
- TNF-$\alpha$-related disorders, and
- cell proliferative disorders.

The pathologic conditions and disorders concerned by the said use, and the corresponding methods of prevention or treatment, are detailed hereinbelow. Any of the uses mentioned with respect to the present invention may be restricted to a non-medical use (e.g. in a cosmetic composition), a non-therapeutic use, a non-diagnostic use, a non-human use (e.g. in a veterinary composition), or exclusively an *in-vitro* use, or a use with cells remote from an animal.

In one embodiment, the present invention further relates to a pharmaceutical composition comprising:

(a) one or more trisubstituted pyrido(2,3-d)pyrimidine derivatives belonging to the class represented by the general formula (I) (or a sub-group thereof), and
(b) one or more pharmaceutically acceptable carriers.

In another embodiment, this invention provides combinations, preferably synergistic combinations, of one or more trisubstituted pyrido(2,3-d)pyrimidine derivatives belonging to the class represented by the general formula (I) (or a sub-group thereof) with one or more biologically-active drugs being preferably selected from the group consisting of immunosuppressant and/or immunomodulator drugs and/or antineoplastic drugs. As is conventional in the art, the evaluation of a synergistic effect in a drug combination may be made by analyzing the quantification of the interactions between individual drugs, using the median effect principle described by Chou et al. in Adv. Enzyme Reg. (1984) 22: 27. Briefly, this principle states that interactions (synergism, additivity, antagonism) between two drugs can be quantified using the combination index (hereinafter referred as CI) defined by the following equation:

$$CI_x = \frac{ED_x^{1c}}{ED_x^{1a}} + \frac{ED_x^{2c}}{ED_x^{2a}}$$

wherein $ED_x$ is the dose of the first or respectively second drug used alone (1a, 2a), or in combination with the second or respectively first drug (1 c, 2c), which is needed to produce a given effect. The said first and second drug have synergistic or additive or antagonistic effects depending upon CI < 1, CI = 1, or CI > 1, respectively. As will be explained in more detail herein below, this principle may be applied to a number of desirable effects such as, but not limited to, an activity against transplant rejection, an activity against immunosuppression or immunomodulation, or an activity against cell proliferation.

For instance the present invention relates to a pharmaceutical composition or combined preparation having synergistic effects against immuno-suppression or immunomodulation and containing:

(a) one or more immunosuppressant and/or immunomodulator drugs, and
(b) at least one trisubstituted pyrido(2,3-d)pyrimidine derivative belonging to the class represented by the general formula (I) (or a sub-group thereof), and
(c) optionally one or more pharmaceutical excipients or pharmaceutically acceptable carriers,

for simultaneous, separate or sequential use in the treatment or prevention of autoimmune disorders and/or in transplant-rejections.

Suitable immunosuppressant drugs for inclusion in the synergistic compositions or combined preparations of this invention belong to a well known therapeutic class. They may be selected, without limitation, from the group consisting of cyclosporin A, substituted xanthines (e.g. methylxanthines such as pentoxyfylline), daltroban, sirolimus, tacrolimus, rapamycin (and derivatives thereof such as defined below), leflunomide (or its main active metabolite A771726, or analogs thereof called malononitrilamides), mycophenolic acid and salts thereof (including the sodium salt marketed under the trade name Mofetil®), adrenocortical steroids, azathioprine, brequinar, gusperimus, 6-mercaptopurine, mizoribine, chloroquine, hydroxy-chloroquine and monoclonal antibodies with immunosuppressive properties (e.g. etanercept, infliximab or kineret). Adrenocortical steroids within the meaning of this invention mainly include glucocorticoids such as but not limited to ciprocinonide, desoxycorticisterone, fludrocortisone, flumoxonide, hydrocortisone, naflocort, procinonide, timobesone, tipredane, dexamethasone, methylprednisolone, methotrexate, prednisone, prednisolone, triamcinolone and pharmaceutically acceptable salts thereof. Rapamycin derivatives as referred herein include O-alkylated derivatives, particularly 9-deoxorapamycins, 26-dihydrorapamycins, 40-O-substituted rapamycins and 28,40-O,O-disubstituted rapamycins (as disclosed in U.S. Patent No. 5,665,772) such as 40-O-(2-hydroxy) ethyl rapamycin - also known as SDZ-RAD -, pegylated rapamycin (as disclosed in U.S. Patent No. 5,780,462), ethers of 7-desmethylrapamycin (as disclosed in U.S. Patent No. 6,440,991) and polyethylene glycol esters of SDZ-RAD (as disclosed in U.S. Patent No. 6,331,547).

Suitable immunomodulator drugs for inclusion into the synergistic immunomodulating pharmaceutical compositions or combined preparations of this invention may be selected, without limitation, from the group consisting of acemannan, amiprilose, bucillamine, dimepranol, ditiocarb sodium, imiquimod, Inosine Pranobex, interferon-β, interferon-γ, lentinan, levamisole, lisophylline, pidotimod, romurtide, platonin, procodazole, propagermanium, thymomodulin, thymopentin and ubenimex.

Synergistic activity of the pharmaceutical compositions or combined preparations of this invention against immunosuppression or immuno-modulation may be readily determined by means of one or more lymphocyte activation tests. Usually activation is measured via lymphocyte proliferation. Inhibition of proliferation thus always means immunosuppression under the experimental conditions applied. There exist different stimuli for lymphocyte activation, in particular:

a) co-culture of lymphocytes of different species (mixed lymphocyte reaction, hereinafter referred as MLR) in a so-called mixed lymphocyte culture test: lymphocytes expressing different minor and major antigens of the HLA-DR type (= alloantigens) activate each other non-specifically;
b) a CD3 assay wherein there is an activation of the T-lymphocytes via an exogenously added antibody (OKT3). This antibody reacts against a CD3 molecule located on the lymphocyte membrane which has a co-stimulatory function. Interaction between OKT3 and CD3 results in T-cell activation which proceeds via the $Ca^{2+}$/calmodulin/calcineurin system and can be inhibited e.g. by cyclosporin A (hereinafter referred as CyA);
c) a CD28 assay wherein specific activation of the T-lymphocyte proceeds via an exogenously added antibody against a CD28 molecule which is also located on the lymphocyte membrane and delivers strong co-stimulatory signals. This activation is $Ca^{2+}$-independent and thus cannot be inhibited by CyA.

Determination of the immunosuppressing or immunomodulating activity of the trisubstituted pyrido(2,3-d)pyrimidine derivatives of this invention, as well as synergistic combinations comprising them, is preferably based on the determination of one or more, preferably two, and more preferably three lymphocyte activation *in vitro* tests, this set of tests most preferably including at least one of the MLR test, CD3 assay and CD28 assay referred above. Preferably the lymphocyte activation *in vitro* tests used include at least two assays for two different clusters of differentiation preferably belonging to the same general type of such clusters and more preferably belonging to type I transmembrane proteins. Optionally the determination of the immuno-suppressing or immunomodulating activity may be performed on the basis of other lymphocyte activation *in vitro* tests, for instance by performing a TNF-$\alpha$ assay or an IL-1 assay or an IL-6 assay or an IL-10 assay or an IL-12 assay or an assay for a cluster of differentiation belonging to a further general type of such clusters and more preferably belonging to type II transmembrane proteins such as, but not limited to, CD69, CD 71 or CD134.

The synergistic effect may be evaluated by the median effect analysis method described herein before. Such tests may for instance, according to standard practice in the art, involve the use of equiment, such as flow cytometer, being able to separate and sort a number of cell subcategories at the end of the analysis, before these purified batches can be analysed further.

Synergistic activity of the pharmaceutical compositions of this invention in the prevention or treatment of transplant rejection may be readily determined by means of one or more leukocyte activation tests performed in a Whole Blood Assay (hereinafter referred as WBA) described for instance by Lin et al. in Transplantation (1997) 63:1734-1738. WBA used herein is a lymphoproliferation assay performed *in vitro* using lymphocytes present in the whole blood, taken from animals that were previously given the disubstituted or trisubstituted pyrido(2,3-d)pyrimidine derivative of this invention, and optionally the other immunosuppressant drug, *in vivo.* Hence this assay reflects the *in vivo* effect of substances as assessed by an *in vitro* read-out assay. The synergistic effect may be evaluated by the median effect analysis method described herein before. Various organ transplantation models in animals are also available *in vivo,* which are strongly influenced by different immunogenicities, depending on the donor and recipient species used and depending on the nature of the transplanted organ. The survival time of transplanted organs can thus be used to measure the suppression of the immune response.

The pharmaceutical composition or combined preparation with synergistic activity against immunosuppression or immunomodulation according to this invention may contain the trisubstituted pyrido(2,3-d)pyrimidine derivative of formula (I) over a broad content range, depending on the specific use being contemplated and upon the expected effect of the preparation on the relevant patient. Usually, the trisubstituted pyrido(2,3-d)pyrimidine derivative content in the combined preparation of the invention is within the range of from 0.1 to 99.9 % by weight, preferably from 1 to 99 % by weight, more preferably from about 5 to 95 % by weight.

The invention further relates to a composition or combined preparation having synergistic effects against cell proliferation and containing:

(a) one or more antineoplastic drugs, and
(b) at least one trisubstituted pyrido(2,3-d)pyrimidine derivative belonging to the class represented by the general formula (I) (or a sub-group thereof), and
(c) optionally one or more pharmaceutical excipients or pharmaceutically acceptable carriers,

for simultaneous, separate or sequential use in the treatment or prevention of cell proliferative disorders.

Suitable antineoplastic drugs for inclusion into the synergistic antiproliferative pharmaceutical compositions or combined preparations of this invention may be, without limitation, selected from the group consisting of alkaloids, alkylating agents (including but not limited to alkyl sulfonates, aziridines, ethylenimines, methylmelamines, nitrogen mustards and nitrosoureas), antibiotics, antimetabolites (including but not limited to folic acid analogues, purine analogs and pyrimidine analogues), enzymes, interferon and platinum complexes. More specific examples include acivicin; aclarubicin; acodazole; acronine; adozelesin; aldesleukin; altretamine; ambomycin; ametantrone; aminoglutethimide; amsacrine; anastrozole; anthramycin; asparaginase; asperlin; azacitidine; azetepa; azotomycin; batimastat; benzodepa; bicalutamide; bisantrene; bisnafide; bizelesin; bleomycin; brequinar; bropirimine; busulfan; cactinomycin; calusterone; caracemide; carbetimer; carboplatin; carmustine; carubicin; carzelesin; cedefingol; chlorambucil; cirolemycin; cisplatin; cladribine; crisnatol; cyclophosphamide; cytarabine; dacarbazine; dactinomycin; daunorubicin; decitabine; dexormaplatin; dezaguanine; diaziquone; docetaxel; doxorubicin; droloxifene; dromostanolone; duazomycin; edatrexate; eflomithine; elsamitrucin; enloplatin; enpromate; epipropidine; epirubicin; erbulozole; esorubicin; estramustine; etanidazole; ethiodized oil $I^{131}$; etoposide; etoprine; fadrozole; fazarabine; fenretinide; floxuridine; fludarabine; fluorouracil; flurocitabine; fosquidone; fostriecin; gemcitabine; Gold 198; hydroxyurea; idarubicin; ifosfamide; ilmofosine; interferon $\alpha$-2a; interferon $\alpha$-2b; interferon $\alpha$-n1; interferon $\alpha$-n3; interferon $\beta$-1a; interferon $\gamma$-1b; iproplatin; irinotecan; lanreotide; letrozole; leuprolide; liarozole; lometrexol; lomustine; losoxantrone; masoprocol; maytansine; mechlorethamine; megestrol; melengestrol; melphalan; menogaril; mercaptopurine; methotrexate; metoprine; meturedepa; mitindomide; mitocarcin;

mitocromin; mitogillin; mitomalcin; mitomycin; mitosper; mitotane; mitoxantrone; mycophenolic acid; nocodazole; nogalamycin; ormaplatin; oxisuran; paclitaxel; pegaspargase; peliomycin; pentamustine; peplomycin; perfosfamide; pipobroman; piposulfan; piroxantrone; plicamycin; plomestane; porfimer; porfiromycin; prednimustine; procarbazine; puromycin; pyrazofurin; riboprine; rogletimide; safingol; semustine; simtrazene; sparfosate; sparsomycin; spirogermanium;, spiromustine; spiroplatin; streptonigrin; streptozocin; strontium 89 chloride; sulofenur; talisomycin; taxane; taxoid; tecogalan; tegafur; teloxantrone; temoporfin; teniposide; teroxirone; testolactone; thiamiprine; thioguanine; thiotepa; tiazofurin; tirapazamine; topotecan; toremifene; trestolone; triciribine; trimetrexate; triptorelin; tubulozole; uracil mustard; uredepa; vapreotide; verteporfin; vinblastine; vincristine; vindesine; vinepidine; vinglycinate; vinleurosine; vinorelbine; vinrosidine; vinzolidine; vorozole; zeniplatin; zinostatin; zorubicin; and their pharmaceutically acceptable salts.

Other suitable anti-neoplastic compounds include vitamin $D_3$ derivatives such as, but not limited to, 20-epi-1,25 dihydroxyvitamin $D_3$; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogens such as, but not limited to, benorterone, cioteronel, cyproterone, delmadinone, oxendolone, topterone, zanoterone and their pharmaceutically acceptable salts; anti-estrogens such as, but not limited to, clometherone; delmadinone; nafoxidine; nitromifene; raloxifene; tamoxifen; toremifene; trioxifene and their pharmaceutically acceptable salts; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin; azasetron; azatoxin; azatyrosine; baccatin III derivatives; balanol; batimastat; BCR/ABL antagonists; benzochlorins; benzoylstaurosporine; β-lactam derivatives; β-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors; castanospermine; cecropin B; cetrorelix; chlorins; chloroquinoxaline sulfonamide; cicaprost; cis-porphyrin; clomifene and analogues thereof; clotrimazole; collismycin A and B; combretastatin and analogues thereof; conagenin; crambescidin 816; cryptophycin and derivatives thereof; curacin A; cyclopentanthraquinones; cycloplatam; cypemycin; cytarabine; cytolytic factor; cytostatin; dacliximab; dehydrodidemnin B; deslorelin; dexifosfamide; dexrazoxane; dexverapamil; didemnin B; didox; diethylnorspermine; dihydro-5-azacytidine; dihydrotaxol; dioxamycin; diphenyl spiromustine; docosanol; dolasetron; doxifluridine; droloxifene; dronabinol; duocarmycin SA; ebselen; ecomustine; edelfosine; edrecolomab; elemene; emitefur; epristeride; estrogen agonists and antagonists; exemestane; filgrastim; finasteride; flavopiridol; flezelastine; fluasterone; fluorodaunorunicin; forfenimex; formestane; fotemustine; gadolinium texaphyrin; gallium nitrate; galocitabine; ganirelix; gelatinase inhibitors; glutathione inhibitors; hepsulfam; heregulin; hexamethylene bisacetamide; hypericin; ibandronic acid; idoxifene; idramantone; ilomastat; imidazoacridones; imiquimod; immunostimulant peptides; insulin-like growth factor-1 receptor inhibitor; interferon agonists; iobenguane; iododoxorubicin; ipomeanol; irinotecan; iroplact; irsogladine; isobengazole; isohomohalicondrin B; itasetron; jasplakinolide; kahalalide F; lamellarin-N; leinamycin; lenograstim; lentinan; leptolstatin; leukemia inhibiting factor; leuprorelin; levamisole; liarozole; lissoclinamide; lobaplatin; lombricine; lonidamine; lovastatin; loxoribine; lurtotecan; lutetium texaphyrin; lysofylline; mannostatin A; marimastat; masoprocol; maspin; matrilysin inhibitors; matrix metalloproteinase inhibitors; merbarone; meterelin; methioninase; metoclopramide; MIF inhibitors; mifepristone; miltefosine; mirimostim; mitoguazone; mitolactol; mitonafide; mitotoxin fibroblast growth factor-saporin; mofarotene; molgramostim; human chorionic gonadotrophin monoclonal antibody; mopidamol; mycaperoxide B; myriaporone; N-acetyldinaline; N-substituted benzamides; nafarelin; nagrestip; naloxone; pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; octreotide; okicenone; onapristone; ondansetron; ondansetron; oracin; osaterone; oxaliplatin; oxaunomycin; palauamine; palmitoylrhizoxin; pamidronic acid; panaxytriol; panomifene; parabactin; pazelliptine; peldesine; pentosan; pentostatin; pentrozole; perflubron; perillyl alcohol; phenazinomycin; phenylacetate; phosphatase inhibitors; picibanil; pilocarpine; pirarubicin; piritrexim; placetin A and B; plasminogen activator inhibitor; propyl bis-acridone; prostaglandin J2; proteasome inhibitors; protein kinase C inhibitors; protein tyrosine phosphatase inhibitors; purine nucleoside phosphorylase inhibitors; purpurins; pyrazoloacridine; raltitrexed; ramosetron; ras farnesyl protein transferase inhibitors; ras inhibitors; ras-GAP inhibitors; retelliptine; rhenium 186 etidronate; rhizoxin; retinamide; rohitukine; romurtide; roquinimex; rubiginone B1; ruboxyl; saintopin; sarcophytol A; sargramostim; sizofiran; sobuzoxane; sodium borocaptate; sodium phenylacetate; solverol; somatomedin binding protein; sonermin; sparfosic acid; spicamycin D; splenopentin; spongistatin 1; squalamine; stem-cell division inhibitors; stipiamide; stromelysin inhibitors; sulfinosine; suradista; suramin; swainsonine; tallimustine; tamoxifen; tauromustine; tazarotene; tecogalan; tellurapyrylium; telomerase inhibitors; temozolomide; tetrachlorodecaoxide; tetrazomine; thaliblastine; thiocoraline; thrombopoietin; thymalfasin; thymopoietin receptor agonist; thymotrinan; thyroid stimulating hormone; tin ethyl etiopurpurin; titanocene; topsentin; tretinoin; triacetyluridine; tropisetron; turosteride; tyrosine kinase inhibitors; tyrphostins; ubenimex; urogenital sinus-derived growth inhibitory factor; urokinase receptor antagonists; variolin B; velaresol; veramine; verdins; verteporfin; vinxaltine; vitaxin; zanoterone; zilascorb; and their pharmaceutically

acceptable salts.

The trisubstituted pyrido(2,3-d)pyrimidine compounds of this invention may also be administered in combination with one or more anti-cancer agents which act by arresting cells in the G2-M phases due to stabilized microtubules. In addition to Taxol (paclitaxel), analogues and derivatives thereof, other examples of anti-cancer agents which act by this mechanism include, without limitation, the following marketed drugs and drugs in development: erbulozole, dolastatin, mivobulin isethionate, discodermolide, altorhyrtins, spongistatins, cemadotin hydrochloride, epothilones desoxyepothilone, 16-aza-epothilone, 21-aminoepothilone, 21-hydroxyepothilone, 26-fluoroepothilone, auristatin, soblidotin, cryptophycin, vitilevuamide, tubulysin, canadensol, centaureidin, oncocidin, fijianolide, laulimalide, narcosine, nascapine, hemiasterlin, vanadocene acetylacetonate, monsatrol, inanocine, eleutherobins, caribaeoside, caribaeolin, halichondrin, diazonamide, taccalonolide, diozostatin, phenylahistin, myoseverin, resverastatin phosphate sodium, and their pharmaceutically acceptable salts.

Synergistic activity of the pharmaceutical compositions or combined preparations of this invention against cell proliferation may be readily determined by means of one or more tests such as, but not limited to, the measurement of the radioactivity resulting from the incorporation of $^3$H-thymidine in culture of tumor cell lines. For instance, different tumor cell lines may be selected in order to evaluate the anti-tumor effects of the test compounds, such as but not limited to:

- RPMI1788: human Peripheral Blood Leucocytes (PBL) Caucasian tumor line,
- Jurkat: human acute T cell leukemia,
- EL4: C57Bl/6 mouse lymphoma, or
- THP-1: human monocyte tumor line.

Depending on the selected tumor cell line, and according to general knowledge in the art, various culture media may be used for such tests, such as for example:

- for RPMI1788 and THP-1: RPMI-1640 + 10% FCS + 1% NEAA + 1% sodium pyruvate + 5x10$^{-5}$ mercapto-ethanol + antibiotics (G-418 0.45 μg/ml).
- for Jurkat and EL4: RPMI-1640 + 10% FCS + antibiotics (G-418 0.45 μg/ml).

In a specific embodiment of the cell proliferation synergy determination test, tumor cell lines are harvested and a suspension of 0.27x10$^6$ cells/ml in whole medium is prepared. The suspensions (150 μl) are added to a microtiter plate in triplicate. Either complete medium (controls) or the test compounds at the test concentrations (50 μl) are added to the cell suspension in the microtiter plate. Cells are incubated at 37°C under 5% $CO_2$ for about 16 hours. $^3$H-thymidine is added, and cells are incubated for another 8 hours and then harvested, and radioactivity is measured in counts per minute (CPM) in a β-counter. The $^3$H-thymidine cell content, and thus the measured radioactivity, is proportional to the proliferation of the cell lines. The synergistic effect is evaluated by the median effect analysis method as disclosed herein before.

The pharmaceutical composition or combined preparation with synergistic activity against cell proliferation according to this invention may contain the trisubstituted pyrido(2,3-d)pyrimidine derivative of the general formula (I) (including any species thereof) over a broad content range depending on the specific use being contemplated and upon the expected effect of the preparation. Generally, the trisubstituted pyrido(2,3-d)pyrimidine derivative content of the combined preparation is within the range of from 0.1 to 99.9 % by weight, preferably from 1 to 99 % by weight, more preferably from about 5 to 95 % by weight.

The pharmaceutical compositions and combined preparations according to this invention may be administered orally or in any other suitable fashion. Oral administration is preferred in many situations, and the corresponding composition or preparation may have the form of a tablet, an aqueous dispersion, a dispersable powder or granule, an emulsion, a hard or soft capsule, a syrup, an elixir or a gel. The dosing forms may be prepared using any method known in the art for manufacturing such pharmaceutical compositions or preparations and may comprise one or more additives such as, but not limited to, sweeteners, flavoring agents, coloring agents, preservatives and the like.

Pharmaceutically acceptable carrier materials and excipients are detailed hereinbelow and may include, *inter alia,* calcium carbonate, sodium carbonate, lactose, calcium phosphate or sodium phosphate; granulating and disintegrating agents, binding agents and the like. The pharmaceutical composition or combined preparation of this invention may be included in a gelatin capsule mixed with any inert solid diluent or carrier material, or has the form of a soft gelatin capsule, in which the ingredient is mixed with a water or oil medium. Aqueous dispersions may comprise the biologically active composition or combined preparation in combination with a suspending agent, dispersing agent or wetting agent. Oil dispersions may comprise suspending agents such as a vegetable oil. Rectal administration is also applicable, for instance in the form of suppositories or gels. Injection (e.g. intramuscularly or intraperiteneously) is also applicable as a mode of administration, for instance in the form of injectable solutions or dispersions, depending upon the disorder to be treated and the condition of the patient.

Auto-immune disorders to be prevented or treated by the pharmaceutical compositions or combined preparations of this invention include both :

- systemic auto-immune diseases such as, but not limited to, lupus erythematosus, psoriasis, vasculitis, polymyositis, scleroderma, multiple sclerosis, ankylosing spondilytis, rheumatoid arthritis and Sjögren syndrome; auto-immune endocrine disorders such as thyroiditis; and
- organ-specific auto-immune diseases such as, but not limited to, Addison disease, hemolytic or pernicious anemia, Goodpasture syndrome, Graves disease, idiopathic thrombocytopenic purpura, insulin-dependent diabetes mellitus, juvenile diabetes, uveitis, Crohn's disease, ulcerative colitis, pemphigus, atopic dermatitis, autoimmune hepatitis, primary biliary cirrhosis, autoimmune pneumonitis, autoimmune carditis, myasthenia gravis, glomerulonephritis and spontaneous infertility.

Transplant rejections to be prevented or treated by the pharmaceutical compositions or combined preparations of this invention include the rejection of transplanted or grafted organs or cells (both allografts and xenografts), such as but not limited to host versus graft reaction disease. The term " organ " as used herein means all organs or parts of organs in mammals, in particular humans, such as but not limited to kidney, lung, bone marrow, hair, cornea, eye (vitreous), heart, heart valve, liver, pancreas, blood vessel, skin, muscle, bone, intestine or stomach. The term " rejection " as used herein means all reactions of the recipient body or the transplanted organ which in the end lead to cell or tissue death in the transplanted organ or adversely affect the functional ability and viability of the transplanted organ or the recipient. In particular, this means acute and chronic rejection reactions. Also included in this invention is preventing or treating the rejection of cell transplants and xenotransplantation. The major hurdle for xenotransplantation is that even before the T lymphocytes, responsible for the rejection of allografts, are activated, the innate immune system, especially T-independent B lymphocytes and macrophages are activated. This provokes two types of severe and early acute rejection called hyper-acute rejection and vascular rejection, respectively. The present invention addresses the problem that conventional immunosuppressant drugs like cyclosporin A are ineffective in xeno-transplantation. The ability of the compounds of this invention to suppress T-independent xeno-antibody production as well as macrophage activation may be evaluated in the ability to prevent xenograft rejection in athymic, T-deficient mice receiving xenogenic hamster-heart grafts.

Cell proliferative disorders to be prevented or treated by the pharmaceutical compositions or combined preparations of this invention include any kind of tumor progression or invasion or metastasis inhibition of a cancer of any type but preferably one selected from the group consisting of lung cancer, leukaemia, ovarian cancer, sarcoma, Kaposi's sarcoma, meningioma, colon cancer, lymp node tumor, glioblastoma multiforme, prostate cancer, bone cancer, testicular cancer, or skin carcinose.

Central nervous system disorders that can be prevented or treated by the pharmaceutical compositions or preparations of this invention include cognitive pathologies such as, but not limited to, dementia, cerebral ischemia, trauma, epilepsy, schizophrenia, chronic pain and neurologic disorders such as but not limited to depression, social phobia and obsessive compulsive disorders.

Cardiovascular disorders to be prevented or treated by the pharmaceutical compositions of this invention include ischemic disorders, infarct or reperfusion damage, atherosclerosis and stroke.

TNF-$\alpha$-related disorders to be prevented or treated by the pharmaceutical compositions of this invention include the following:

- septic or endotoxic shock or sepsis, especially in patients with a serum level of interleukin-6 above 1,000 pg/ml at start of treatment;
- vascular TNF-$\alpha$- mediated diseases such as, but not limited to, disseminated intravascular coagulation and Kawasaki's pathology;
- pathologies and conditions associated with and/or induced by abnormal levels of TNF-$\alpha$ (herein defined as exceeding by at least 10 % and at most 500 % the TNF-$\alpha$ level present in a normal healthy subject) occurring in a systemic, localized or particular tissue type or location in the body of the mammal; such tissue types include, but are not limited to, blood, lymph, liver, kidney, spleen, heart muscle or blood vessels, brain or spinal cord white matter or grey matter, cartilage, ligaments, tendons, lung, pancreas, ovary, testes and prostate. Abnormal TNF-$\alpha$ levels can also be localized to specific regions or cells in the body, such as joints, nerve blood vessel junctions and bones. Such pathologies include alcohol-induced hepatitis; neurodegenerative diseases such as extrapyramidal and cerebellar disorders including lesions of the corticospinal system; disorders of the basal ganglia; hyperkinetic movement disorders such as chorea; drug-induced movement disorders; hypokinetic movement disorders, such as Parkinson's disease; spinocerebellar degenerations such as spinal ataxia, multiple systems degenerations (including Dejerine-Klumpke syndrome) and systemic disorders (including Refsum's disease, abetalipoprotemia, ataxia and telangiectasia); disorders of the motor unit, such as neurogenic muscular atrophies (anterior horn cell degeneration, such as amyotrophic

lateral sclerosis, infantile spinal muscular atrophy and juvenile spinal muscular atrophy); Alzheimer's disease; Wernicke-Korsakoff syndrome; Creutzfeldt-Jakob disease; Hallerrorden-Spatz disease; and primary or secondary myelodysplastic syndromes;

- toxic effects of TNF-$\alpha$ and/or anti-cancer chemotherapeutic agents, especially side effects associated with TNF-$\alpha$ generation during neoplastic therapy, for instance following use of cisplatin;
- injuries after irradiation of a tissue of a mammal (e.g. a human being) by radio-elements such as, but not limited to, radiation-induced graft-versus-host disease; and
- cachexia and similar chronic wasting diseases, whether associated with cancer or with other chronic diseases such as, but not limited to, malabsortive disorders, excessive physical stress, eating disorders, and AIDS.

The medicament, in this embodiment of the invention may be for prophylactic use, i.e. where circumstances are such that an elevation in the TNF-$\alpha$ level might be expected, or alternatively may be for use in reducing the TNF-$\alpha$ level after it has reached an undesirably high level (as defined herein above) or at the time when TNF-$\alpha$ level is rising in the patient.

The term " pharmaceutically acceptable carrier or excipient " as used herein in relation to pharmaceutical compositions and combined preparations means any material or substance with which the active principle, i.e. the trisubstituted pyrido(2,3-d)pyrimidine derivative of the general formula (I), and optionally the immunosuppressant or immunomodulator or antineoplastic drug, may be formulated in order to facilitate its application or dissemination to the locus to be treated, for instance by dissolving, dispersing or diffusing the said composition, and / or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, pellets or powders.

Suitable pharmaceutical carriers for use in the said pharmaceutical compositions and their formulation are well known to those skilled in the art. There is no particular restriction to their selection within the present invention although, due to the usually low or very low water-solubility of the trisubstituted pyrido(2,3-d)pyrimidine derivatives of this invention, special attention will be paid to the selection of suitable carrier combinations that can assist in properly formulating them in view of the expected time release profile. Suitable pharmaceutical carriers include additives such as, but not limited to, wetting agents, dispersing agents, stickers, adhesives, emulsifying or surface-active agents, thickening agents, complexing agents, gelling agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, dissolving, spray-drying, coating, freeze-drying, extruding, milling and/or grinding the biologically active ingredient (s), in a one-step or a multi-steps procedure, with the selected carrier material(s) and, where appropriate, the other additives such as surface-active agents. may also be prepared by micronisation, for instance in view to obtain them in the form of microspheres usually having a diameter of about 1 to 10 $\mu$m, namely for the manufacture of microcapsules for controlled or sustained release of the biologically active ingredient(s).

Suitable surface-active agents to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps include, but are not limited to, alkaline or alkaline-earth metal salts, unsubstituted or substituted ammonium salts of higher fatty acids ($C_{10}$-$C_{22}$), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the correspon-ding phosphates, e.g. salts of phosphoric acid ester and an adduct of p-nonylphenol with ethylene and / or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanyl-phosphatidylcholine, dipalmitoylphoshatidylcholine and their mixtures in all proportions.

Suitable non-ionic surfactants include, but are not limited to, polyethoxylated and polypropoxylated derivatives of alkylphenols, fatty alcohols, fatty acids, aliphatic amines or amides containing at least 12 carbon atoms in the molecule,

alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediamino-polypropylene glycol containing 1 to 10 carbon atoms in the alkyl chain, which adducts contain 20 to 250 ethyleneglycol ether groups and / or 10 to 100 propyleneglycol ether groups. Such compounds usually contain from 1 to 5 ethyleneglycol units per propyleneglycol unit. Representative examples of non-ionic surfactants are nonylphenol-polyethoxyethanol, castor oil polyglycolic ethers, polypropylene/ polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include, but are not limited to, quaternary ammonium salts, preferably halides, having four hydrocarbon radicals optionally substituted with halo, phenyl, substituted phenyl or hydroxy; for instance quaternary ammonium salts containing as N-substituent at least one $C_8$-$C_{22}$ alkyl radical (e.g. cetyl, lauryl, palmityl, myristyl, oleyl and the like) and, as further sub-stituents, unsubstituted or halogenated lower alkyl, benzyl and / or hydroxy-$C_{1-4}$ alkyl radicals.

A more detailed description of surface-active agents suitable for this purpose may be found for instance in " McCutcheon's Detergents and Emulsifiers Annual " (MC Publishing Crop., Ridgewood, New Jersey, 1981), " Tensid-Taschenbuch ", 2nd ed. (Hanser Verlag, Vienna, 1981) and " Encyclopaedia of Surfactants (Chemical Publishing Co., New York, 1981).

Structure-forming, thickening or gel-forming agents may also be included into the pharmaceutical compositions and combined preparations of the invention. Suitable such agents in particular include, but are not limited to, highly dispersed silicic acid such as a product commercially available under the trade name Aerosil; bentonites; tetraalkyl ammonium salts of montmorillonites (e.g., products commercially available under the trade name Bentone), wherein each of the alkyl groups may contain from 1 to 20 carbon atoms; cetostearyl alcohol and modified castor oil products (e.g. the product commercially available under the trade name Antisettle).

Gelling agents which may also be included into the pharmaceutical compositions and combined preparations of the present invention include, but are not limited to, cellulose derivatives such as carboxymethylcellulose, cellulose acetate and the like; natural gums such as arabic gum, xanthum gum, tragacanth gum, guar gum and the like; gelatin; silicon dioxide; synthetic polymers such as carbomers, and mixtures thereof in all proportions. Gelatin and modified celluloses represent a preferred class of gelling agents.

Other optional excipients which may be included in the pharmaceutical compositions and combined preparations of the present invention include additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium metabisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylene-diamine tetraacetic acid; flavoring agents such as natural vanillin; buffers such as citric acid and acetic acid; extenders or bulking agents such as silicates, diatomaceous earth, magnesium oxide or aluminum oxide; densification agents such as magnesium salts; and mixtures thereof.

Additional ingredients may be included in order to control the duration of action of the biologically-active ingredient in the compositions and combined preparations of the invention. Control release compositions may thus be achieved by selecting appropriate polymer carriers such as for example polyesters, polyamino-acids, polyvinyl-pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethyl-cellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition or combined preparation of the invention may also require protective coatings.

Pharmaceutical forms suitable for injectable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol, complexing agents such as cyclodextrins and the like, and mixtures thereof.

Since, in the case of combined preparations including the trisubstituted pyrido(2,3-d)pyrimidine derivatives of this invention and an immunosuppressant or immunomodulator or antineoplastic drug, both ingredients do not necessarily bring out their synergistic therapeutic effect directly at the same time in the patient to be treated, the said combined preparation may be in the form of a medical kit or package containing the two ingredients in separate but adjacent form. In the latter context, each ingredient may therefore be formulated in a way suitable for an administration route different from that of the other ingredient, e.g. one of them may be in the form of an oral or parenteral formulation whereas the

other is in the form of an ampoule for intravenous injection or an aerosol.

The present invention further relates to a method for preventing or treating a disease selected from the group consisting of central nervous system disorders, cell proliferative disorders, immune and auto-immune disorders, transplant rejections and TNF-α-related disorders in a patient, preferably a mammal, more preferably a human being. The method of this invention consists of administering to the patient in need thereof an effective amount of a trisubstituted pyrido(2,3-d)pyrimidine derivative having the general formula (I), optionally together with an effective amount of another immunosuppressant or immunomodulator or antineoplastic drug, or a pharmaceutical composition comprising the same, such as disclosed above in extensive details. The effective amount is usually in the range of about 0.01 mg to 20 mg, preferably about 0.1 mg to 5 mg, per day per kg bodyweight for humans. Depending upon the pathologic condition to be treated and the patient's condition, the said effective amount may be divided into several sub-units per day or may be administered at more than one day intervals. The patient to be treated may be any warm-blooded animal, preferably a mammal, more preferably a human being, suffering from said pathologic condition.

The present invention will be further described with reference to certain more specific embodiments and examples, but the present invention is not limited thereto but only by the attached claims. The following examples are given by way of illustration only.

Example 1 - synthesis of 2-amino-4-isopropoxy-6-bromo-pyrido[2,3-d]pyrimidine

Sodium (65 mg, 2.82 mmole) was dissolved in isopropanol (30 ml) at 60°C, then 2-pivaloylamino-4-isopropoxy-6-bromo-pyrido[2,3-d]pyrimidine (2.56 mmole, 940 mg) (for instance prepared and characterized according to Taylor et al. in Heterocycles (1993) 36:1889) was added. The reaction mixture was stirred at 70°C for 6 hours, and then acidified with acetic acid (160 μl) and extracted with dichloromethane. The organic layers were evaporated *in vacuo* and the residue was purified by silica gel column chromatography, the mobile phase consisting of a $CH_3OH/CH_2Cl_2$ mixture (in a ratio gradually ranging from 1:99 to 4:96), resulting in the pure title compound (515 mg, yield 71 %) which was characterized by its mass spectrum as follows: MS (m/z): 305, 307 ($[M+Na]^+$, 45), 283, 285 ($[M+H]^+$, 60), 241, 243 ($[M+H\text{-propene})^+$, 100).

Example 2 - synthesis of 2-amino-4-isopropoxy-6-(3,4-dimethoxyphenyl)-pyrido[2,3-d]pyrimidine

To a solution of 2-amino-4-isopropoxy-6-bromo-pyrido[2,3-d]pyrimidine (0.7 mmole, 199 mg) in tetrahydrofuran (15 ml) was added 3,4-dimethoxyphenyl boronic acid (192 mg, 1.054 mmole), tetrakis(triphenylphosphine)palladium(0) (0.0352 mmole, 41 mg) and 15 ml of a 0.4 M $Na_2CO_3$ solution in water. The reaction mixture was refluxed for 16 hours. The solvents were evaporated *in vacuo* and the resulting residue was purified by silica gel column chromatography, the mobile phase being a $CH_3OH/CH_2Cl_2$ mixture (in a ratio gradually ranging from 1:99 to 3:97), resulting in the pure title compound (98 mg, yield 41 %) which was characterized by its mass spectrum as follows: MS (m/z): 703 ($[2M+Na]^+$, 100), 363 ($[M+Na]^+$, 25), 341 ($[M+H]^+$, 100), 299 ($[M+H\text{-propene}]^+$, 90).

Example 3 - synthesis of 2-pivaloylamino-4-morpholino-6-bromo-pyrido[2,3-d]pyrimidine

To a suspension of 2-pivaloylamino-4-(1,2,4-triazolyl)-6-bromo-pyrido[2,3-d]pyrimidine (1.77 g, 4.71 mmole), for instance prepared and characterized according to Taylor et al. in *Heterocycles* (1993) 36:1889, in 1,4-dioxane (100 ml) was added morpholine (492 μl, 5.65 mmole). The suspension became a yellow solution after 1 h and was further stirred at room temperature overnight. Water was added to the reaction mixture and the reaction mixture was extracted with dichloromethane (3 times). The organic layers were evaporated *in vacuo* and the crude residue was purified by silica gel column chromatography, the mobile phase being a $CH_3OH/CH_2Cl_2$ mixture (in a ratio gradually ranging from 3:97 to 6:94), yielding the pure title compound as a white powder (1.37 g, 74 %) which was characterized by its mass spectrum as follows: MS (m/z): 393, 395 ($[M+H]^+$, 100).

Example 4 - synthesis of 2-amino-4-morpholino-6-(3,4-dimethoxyphenyl)-pyrido[2,3-d]pyrimidine

To a solution of 2-pivaloylamino-4-morpholino-6-bromo-pyrido[2,3-d]pyrimidine (650 mg, 1.65 mmole) in tetrahydrofuran (20 ml) was added 3,4-dimethoxyphenyl boronic acid (450 mg, 2.48 mmole), tetrakis(triphenylphosphine) palladium(0) (95 mg, 0.0825 mmole) and 20 ml of a 0.4 M $Na_2CO_3$ solution in water. The reaction mixture was refluxed for 16 hours. The solvents were evaporated *in vacuo* and the resulting residue consisted of a mixture of 2-amino-4-morpholino-6-(3,4-dimethoxyphenyl)-pyrido[2,3-d]pyrimidine and 2-pivaloylamino-4-morpholino-6-(3,4-dimethoxy-phenyl)-pyrido[2,3-d]pyrimidine. This residue was resuspended in a mixture of methanol (20 ml) and a 20 % $K_2CO_3$ solution in water (20 ml). The reaction mixture was then refluxed for 2 hours, after which no pivaloylated material could be observed on thin layer chromatography. The solvents were evaporated *in vacuo* and the residue was purified by silica

gel column chromatography, the mobile phase consisting of a $CH_3OH/CH_2Cl_2$ mixture (in a ratio gradually ranging from 2:98 to 3:97), resulting in the title compound as a white powder (212 mg, yield 35 %) which was characterized by its mass spectrum as follows: MS (m/z): 368 ($[M+H]^+$, 100).

Example 5 - synthesis of 2-pivaloylamino-4-*N*-piperazino-6-bromo-pyrido[2,3-d]pyrimidine

To a suspension of 2-pivaloylamino-4-(1,2,4-triazolyl)-6-bromo-pyrido[2,3-d] pyrimidine (1.902 g, 5.05 mmole), for instance prepared and characterized according to Taylor et al. in *Heterocycles* (1993) 36:1889, in 1,4-dioxane (110 ml) was added piperazine (523 mg, 6.07 mmole). The suspension became a yellow solution after 1 hour and was further stirred at room temperature for 16 hours. Water was added to the reaction mixture, which was then extracted three times with dichloromethane. The organic layers were evaporated *in vacuo* and the crude residue was purified by silica gel column chromatography, the mobile phase being a $CH_3OH/CH_2Cl_2$ mixture (in a ratio of 6:94 with 0.5 % concentrated aqueous ammonia), resulting in the pure title compound as a white powder (969 mg, yield 62 %) which was characterized by its mass spectrum as follows: MS (m/z): 393, 395 ($[M+H]^+$, 100).

Example 6 - synthesis of 2-pivaloylamino-4-[N-4-methylphenylcarbamoyl-piperazin-1-yl]-6-bromo-pyrido[2,3-d]pyrimidine

To a solution of 2-pivaloylamino-4-*N*-piperazino-6-bromo-pyrido[2,3-d]pyrimidine (1.18 g, 3.75 mmole) in dichloromethane (25 ml) was added p-tolyl isocyanate (500 mg, 3.75 mmole). The mixture was stirred at room temperature for 1 hour. The solvents were evaporated *in vacuo* and the residue was purified by silica gel column chromatography, the mobile phase being a mixture of $CH_3OH$ and $CH_2Cl_2$ (in a ratio of 1:30), resulting in the title compound as a yellowish powder (1.32 g, yield 84 %) which was characterized by its mass spectrum as follows: MS (m/z): 526.1, 528.2 ($[M+H]^+$, 100).

Example 7 - synthesis of 2-amino-4-[N-4-methylphenylcarbamoyl-piperazin-1-yl]-6-bromo-pyrido[2,3-d]pyrimidine

A solution of 2-pivaloylamino-4-[*N*-4-methylphenylcarbamoyl-piperazin-1-yl]-6-bromo-pyrido[2,3-d]pyrimidine (526 mg, 1.0 mmole) and $K_2CO_3$ (415 mg, 3.0 mmole) in MeOH (25 ml) and water (15 ml) was stirred at 50°C for 3 hours. The reaction mixture was extracted with dichloromethane. The organic layers were evaporated *in vacuo* and the crude residue was purified by silica gel column chromatography, the mobile phase being a mixture of $CH_3OH$ and $CH_2Cl_2$ (in a ratio of 1:30), resulting in the title compound as a white solid (370 mg, yield 84 %) which was characterized by its mass spectrum and its ultraviolet light spectrum as follows:

- UV ($MeOH/H_2O$): 222.2, 236.5 and 364.7 nm; and
- MS (m/z): 442.1, 444.1 ($[M+H]^+$, 100).

Example 8 - synthesis of 2-pivaloylamino-4-*N*-piperazinyl-6-(3,4-dimethoxyphenyl)-pyrido[2,3-d]pyrimidine

To a solution of 2-pivaloylamino-4-*N*-piperazino-6-bromo-pyrido[2,3-d]pyrimidine (393 mg, 1.0 mmole) in dioxane (20 ml) and water (5 ml) was added 3,4-dimethoxyphenyl boronic acid (182 mg, 1 mmole), $K_2CO_3$ (550 mg, 3 mmole) and tetrakis(triphenylphosphine)palladium(0) (58 mg, 0.05 mmole). The reaction mixture was heated at 90°C for 1 hour. The reaction mixture was then extracted with dichloromethane and the organic phase was concentrated under reduced pressure. The residue was purified by silica gel flash chromatography, the mobile phase being a mixture of $CH_3OH$ and $CH_2Cl_2$ (ratio 1:15), resulting in the title compound as a yellowish solid (340 mg, yield 76 %) which was characterized by its mass spectrum as follows: MS (m/z): 451.3 ($[M+H]^+$, 100).

Example 9 - synthesis of 2-pivaloylamino-4-[(N-phenoxyacetyl)-N-piperazin-N-yl]-6-(3,4-dimethoxyphenyl)-pyrido[2,3-d]pyrimidine

To a solution of 2-pivaloylamino-4-*N*-piperazine-6-(3,4-dimethoxyphenyl)-pyrido[2,3-d]pyrimidine (180 mg, 0.4 mmole) in pyridine (5 ml) was added phenoxyacetyl chloride (102 mg, 0.6 mmole). The mixture was stirred at room temperature for 20 minutes. The reaction was quenched with water (50 μl). The solvents were evaporated *in vacuo* and the residue was purified by silica gel column chromatography, the mobile phase being a $CH_3OH$ and $CH_2Cl_2$ mixture (ratio 1:40), resulting in the title compound as a yellowish solid (198 mg, yield 85 %) which was characterized by its mass spectrum as follows: MS (m/z): 607.3 ($[M+Na]^+$, 30), 585.4 ($[M+H]^+$, 100).

Example 10 - synthesis of 2-amino-4-(*N*-phenoxyacetyl-*N*-piperazin-1-yl)-6-(3,4-dimethoxyphenyl)pyrido[2, 3d]pyrimidine

A solution of 2-pivaloylamino-4-[(*N*-phenoxyacetyl)-*N*-piperazin-1-yl]-6-(3,4-dimethoxyphenyl)-pyrido[2,3-d] pyrimidine (190 mg, 0.32 mmole) and $K_2CO_3$ (225 mg, 1.6 mmole) in MeOH (15 ml) and water (10 ml) was stirred at 50°C for 4 hours. The reaction mixture was extracted with dichloromethane. The organic layers were evaporated *in vacuo* and the crude residue was purified by silica gel column chromatography, the mobile phase being a 1:20 $CH_3OH/CH_2Cl_2$ mixture, resulting in the title compound as a yellowish solid (130 mg, yield 81 %) which was characterized by its mass spectrum and its ultraviolet light spectrum as follows:

- UV (MeOH/$H_2O$): 216.5, 276.7 and 374.3 nm; and
- MS (m/z): 501.2 ($[M+H]^+$, 100).

Example 11 - synthesis of 2-pivaloylamino-4-[N-4-methylphenylcarbamoyl-piperazin-1-yl]-6-(3,4-dimethoxyphenyl)pyrido[2,3-d]pyrimidine

To a solution of 2-pivaloylamino-4-(*N*-4-methylphenylcarbamoyl-piperazin-1-yl]-6-bromo-pyrido[2,3-d]pyrimidine (1.32 g, 2.5 mmole) in dioxane (50 ml) and water (15 ml) was added 3,4-dimethoxyphenyl boronic acid (455 mg, 2.5 mmole), $K_2CO_3$ (860 mg, 6.2 mmole) and tetrakis(triphenylphosphine)palladium(0) (140 mg, 0.12 mmole). The reaction mixture was heated at 90°C for 1 hour. The reaction mixture was extracted with dichloromethane and the organic phase was concentrated under reduced pressure. The residue was purified by silica gel flash chromatography, the mobile phase being a 1:35 $CH_3OH/CH_2Cl_2$ mixture, resulting in the title compound as a yellow solid (1.31 g, yield 90 %) which was characterized by its mass spectrum as follows: MS (m/z): 584.3 ($[M+H]^+$, 100).

Example 12 - synthesis of 2-amino-4-(*N*-4-methylphenylcarbamoyl-piperazin-1-yl]-6-(3,4-dimethoxyphenyl)pyrido [2,3-d]pyrimidine

A suspension of 2-pivaloylamino-4-(*N*-4-methylphenylcarbamoyl-piperazin-1-yl]-6-(3,4-dimethoxyphenyl)-pyrido[2,3-d]pyrimidine (380 mg, 0.65 mmole) and $K_2CO_3$ (270 mg, 2.0 mmole) in MeOH (25 ml) and water (15 ml) was stirred at 50°C for 3 hours. The reaction mixture was extracted with dichloromethane. The organic layers were evaporated *in vacuo* and the crude residue was purified by silica gel column chromatography, the mobile phase being a 1:30 $CH_3OH/CH_2Cl_2$ mixture, resulting in the title compound as a yellowish solid (215 mg, yield 66 %) which was characterized by its mass spectrum and its ultraviolet light spectrum as follows:

- UV (MeOH/$H_2O$) 222.2, 236.5, 364.7 nm; and
- MS (m/z): 500.3 ($[M+H]^+$, 100).

Example 13 - synthesis of 2-pivaloylamino-4-[(N-benzyloxycarbonyl)-piperazin-1-yl]-6-bromo-pyrido[2, 3-d]pyrimidine

To a solution of 2-pivaloylamino-4-*N*-piperazino-6-bromo-pyrido[2,3-d]pyrimidine (393 mg, 1 mmole) in dichloromethane (20 ml) was added triethylamine (3 mmole) and benzyl chloroformate (1.25 mmole). The reaction mixture was stirred at room temperature for 1 hour. The reaction was quenched with water (200 $\mu$l). The solvents were evaporated *in vacuo* and the residue was purified by silica gel column chromatography, the mobile phase being a 1:30 $CH_3OH/CH_2Cl_2$ mixture, resulting in the title compound as a yellowish solid (400 mg, yield 76 %) which was characterized by its mass spectrum as follows: MS (m/z): 549.2, 551.1($[M+Na]^+$, 35), 527.2, 529.2 ($[M+H]^+$, 50).

Example 14 - synthesis of 2-pivaloylamino-4-[(*N*-benzyloxycarbonyl)-piperazin-1-yl] 6-(3,4-dimethoxyphenyl)-pyrido [2,3-d]pyrimidine

To a solution of 2-pivaloylamino-4-[(*N*-benzyloxycarbonyl)-piperazin-1-yl]-6-bromo-pyrido[2,3-d]pyrimidine (400 mg, 0.76 mmole) in dioxane (20 ml) and water (5 ml) were added 3,4-dimethoxyphenyl boronic acid (170 mg, 0.93 mmole), $K_2CO_3$ (320 mg, 2.3 mmole) and tetrakis(triphenylphosphine)palladium(0) (50 mg, 0.05 mmole). The reaction mixture was heated at 90°C for 30 minutes. The reaction mixture was extracted with dichloromethane and the organic phase was concentrated under reduced pressure. The residue was purified by silica gel flash chromatography, the mobile phase being a 1:30 $CH_3OH/CH_2Cl_2$ mixture, resulting in the title compound as a yellowish solid (420 mg, 94%) which was characterized by its mass spectrum as follows: MS (m/z): 585.3 ($[M+H]^+$, 100).

Example 15 - synthesis of 2-amino-6-(3,4-dimethoxyphenyl)-4-[(*N*-benzyloxycarbonyl)-piperazin-1-yl]-pyrido[2,3-d]pyrimidine

A suspension of 2-pivaloylamino-4-[(*N*-benzyloxycarbonyl)-piperazin-1-yl]-6-(3,4-dimethoxyphenyl) pyrido [2,3-d]pyrimidine (420 mg, 0.72 mmole) and $K_2CO_3$ (300 mg, 2.2 mmole) in MeOH (25 ml) and water (15 ml) was stirred at 50°C for 3 hours. The reaction mixture was extracted with dichloromethane. The organic layers were evaporated *in vacuo* and the crude residue was purified by silica gel column chromatography, the mobile phase being a $CH_3OH/CH_2Cl_2$ mixture 1:30), resulting in the pure title compound as a yellowish solid (180 mg, yield 50 %) which was characterized by its mass spectrum and its ultraviolet light spectrum as follows:

- UV (MeOH/$H_2O$): 214.1, 281.4, 379.1 nm; and
- MS (m/z): 501.3 ($[M+H]^+$, 100).

Examples 16 to 18 - synthesis of 2-pivaloylamino-4-isopropoxy-6-aryl-pyrido[2,3-d]pyrimidines and 2-pivaloylamino-4-isopropoxy-6-heteroaryl-pyrido[2,3-d]pyrimidines

To a solution of 2-pivaloylamino-4-isopropoxy-6-bromo-pyrido[2,3-d]pyrimidine(1 mmole) in dioxane (20 ml) and water (5 ml) was added an appropriate aryl boronic acid (1 mmole), $K_2CO_3$ (3 mmole) and tetrakis(triphenylphosphine) palladium(0) (0.05 mmole). The mixture was heated at 90°C for 2 hours (example 16) or 30 minutes (examples 17 and 18). The reaction mixture was diluted with $CH_2Cl_2$ (50 ml) and the organic phase was concentrated under reduced pressure, resulting in the following crude compounds which were not purified, but later used as such:

- 2-pivaloylamino-4-isopropoxy-6-(4-dimethylaminophenyl)pyrido[2,3-d]pyrimidine (example 16) was obtained from 4-dimethylaminophenyl boronic acid,
- 2-pivaloylamino-4-isopropoxy-6-(4-trifluoromethylphenyl)pyrido[2,3-d]pyrimidine (example 17) was obtained from 4-trifluoromethylphenyl boronic acid; and
- 2-pivaloylamino-4-isopropoxy-6-(2-thienyl)pyrido[2,3-d]pyrimidine (example 18) was obtained from 2-thienyl boronic acid.

Examples 19 to 21 - synthesis of 2-amino-4-isopropoxy-6-aryl-pyrido[2,3-d]pyrimidines and 2-amino-4-isopropoxy-6-heteroaryl-pyrido[2,3-d]pyrimidines

To a suspension of the crude 2-pivaloylamino-4-isopropoxy-6-aryl-pyrido[2,3-d]pyrimidine from examples 16-18 (1 mmole) in MeOH (40 ml) was added $Fe(NO_3)_3$ $9H_2O$ (0.2 mmole). The mixture was stirred at room temperature for 48 hours. After concentration under reduced pressure, the residue was purified by silica gel flash chromatography, the mobile phase being a 1:30 $CH_3OH/CH_2Cl_2$ mixture, resulting in the pure following compounds which were characterized by their mass spectrum and their ultraviolet light spectrum as follows:

- 2-amino-4-isopropoxy-6-(4-dimethylaminophenyl)pyrido[2,3-d]pyrimidine (example 19) was obtained from 2-pivaloylamino-4-isopropoxy-6-(4-dimethylaminophenyl)-pyrido[2,3-d]pyri-midine in 71 % yield as a yellowish solid. UV (MeOH/$H_2O$, nm): 227.0, 315.9, 379.1; MS (m/z): 324.0 ($[M+H]^+$, 100);
- 2-amino-4-isopropoxy-6-(4-trifluoromethylphenyl)-pyrido[2,3-d]pyrimidine (example 20) was obtained from 2-pivaloylamino-4-isopropoxy-6-(4-trifluoromethylphenyl)pyrido[2,3-d]pyri-midine in 52 % yield as a yellowish solid. UV (MeOH/$H_2O$ nm): 289.8, 241.2, 351.8; MS (m/z): 349.0 ($[M+H]^+$, 100); and
- 2-amino-4-isopropoxy-6-(2-thienyl)pyrido[2,3-d]pyrimidine (example 21) was obtained from 2-pivaloylamino-4-isopropoxy-6-(2-thienyl)-pyrido[2,3-d]pyrimidine in 53 % yield as a yellowish solid. UV (MeOH/$H_2O$ nm): 223.5, 306.4, 367.1; MS (m/z): 287.0 ($[M+H]^+$, 100).

Examples 22 and 23 - synthesis of 2-amino-4-substituted-6-(3,4-dimethoxyphenyl)-pyrido[2,3-d]pyrimidines

To a suspension of 2-pivaloylamino-6-(3,4-dimethoxyphenyl)-pyrido[2,3-d]pyrimidin-4-(3H)-one (1 mmole) in toluene (4 ml) was added 1,1,1,3,3,3-hexamethyldisilazane (1 ml, 4.7 mmole), an appropriate amine (4 mmole), *p*-toluenesulfonic acid (20 mg, 0.1 mmole) and ammonium sulphate (20 mg, 0.15 mmole). The reaction mixture was stirred under reflux for 48 hours. The solvents were removed under reduced pressure and the residue was purified by silica gel flash chromatography, the mobile phase being a 1:40 $CH_3OH/CH_2Cl_2$ mixture, to yield the pure following compounds which were characterized by their mass spectrum and their ultraviolet light spectrum as follows:

- 2-amino-4-(piperazin-1-yl)-6-(3,4-dimethoxyphenyl)-pyrido[2,3-d]pyrimidine (example 22) was obtained from piperazine in 68 % yield as a white solid. UV ($MeOH/H_2O$, nm): 216.5, 276.7, 377.9; MS (m/z): 367.2 ($[M+H]^+$, 100); and

- 2-amino-4-(4-benzylpiperazin-1-yl)-6-(3,4-dimethoxyphenyl)-pyrido[2,3-d]pyrimidine (example 23) was obtained from 1-benzylpiperazine in 26 % yield as a white solid. UV ($MeOH/H_2O$, nm): 215.3, 280.3, 375.5; MS (m/z): 357.3 ($[M+H]^+$, 100).

Example 24 - synthesis of 2-pivaloylamino-6-(3,4-dimethoxyphenyl)pyrido[2,3-d]pyrimidin-4-(3H)-one and 2-pivaloylamino-4-(1,2,4-triazolyl)-6-(3,4-dimethoxyphenyl) pyrido[2,3-d]pyrimidine

To a solution of 2-pivaloylamino-6-bromo-pyrido[2,3-d]pyrimidin-4-(3H)one (210 mg, 0.65 mmole), synthesized and characterised according to Taylor et al. in Heterocycles (1993) 36 :1883, in dioxane (20 ml) and water (8 ml) was added 3,4-dimethoxyphenyl boronic acid (0.65 mmol, 118 mg,) $K_2CO_3$ (223 mg, 1.6 mmole) and tetrakis(triphenylphosphine)palladium(0) (38 mg, 0.032 mmole). The reaction mixture was heated for 2 hours at 90°C. The reaction mixture was extracted with dichloromethane and the organic phase was concentrated under reduced pressure. The residue was purified by silica gel flash chromatography, the mobile phase being a 3:97 $CH_3OH/CH_2Cl_2$ mixture, resulting in 2-pivaloylamino-6-(3,4-dimethoxyphenyl)pyrido[2,3-d]pyrimidin-4-(3H)-one (155 mg, yield : 63 %), characterized by its mass spectrum as follows : MS (m/z) : 383 ($[M+H]^+$, 100)

To a solution of 2-pivaloylamino-6-(3,4-dimethoxyphenyl)pyrido[2,3-d]pyrimidin-4-(3H)-one (1.0 mmole) in acetonitrile (20 ml) were added 1,2,4-triazole (10 mmole), triethylamine (3 mmole) and $POCl_3$ (3 mmole). The reaction mixture was stirred at room temperature for 24 hours (with thin layer chromatography monitoring). The reaction mixture was diluted with $CHCl_3$ (30 ml) and washed with a 5 % aqueous hydrochloric acid solution. The organic layer was concentrated under reduced pressure yielding crude 2-pivaloylamino-4-(1,2,4-triazolyl)-6-(3,4-dimethoxyphenyl) pyrido [2,3-d]pyrimidine, which was used for further reaction without any purification.

Examples 25 to 27 - synthesis of 2-pivaloylamino-4-substituted-6-(3,4-dimethoxyphenyl)pyrido[2,3-d]pyrimidines

The crude 2-pivaloylamino-4-(1,2,4-triazolyl)-6-(3,4-dimethoxyphenyl)-pyrido[2,3-d]pyrimidine from example 24 was dissolved in dioxane (20 ml), and an appropriate primary amine (2-3 equivalents) was added. The mixture was stirred at room temperature for 12 hours. The solvents were evaporated *in vacuo* yielding the following crude compounds, which were used for further reaction without any purification:

- 2-pivaloylamino-4-*n*-propylamino-6-(3,4-dimethoxyphenyl)pyrido[2,3-d]pyrimidine (example 25) was obtained from n-propylamine;
- 2-pivaloylamino-4-isopropylamino-6-(3,4-dimethoxyphenyl)pyrido[2,3-d]pyrimidine (example 26) was obtained from isopropylamine; and
- 2-pivaloylamino-4-(1-piperidino)-6-(3,4-dimethoxyphenyl)pyrido[2,3-d]pyrimidine (example 27) was obtained from piperidine.

Examples 28 and 29 - synthesis of 2-pivaloylamino-4-substituted-6-(3,4-dimethoxyphenyl)pyrido[2,3-d]pyrimidine

Sodium (3 mmole) was dissolved in an appropriate alcohol (10 mmole) and THF (30 ml) by refluxing. After cooling to room temperature, the crude 2-pivaloyl-amino-4-(1,2,4-triazolyl)-6-(3,4-dimethoxyphenyl)pyrido[2,3-d]pyrimidine (1 mmole) from example 24 was added. The mixture was stirred at room temperature for 30 minutes. The reaction mixture was diluted with $CH_2Cl_2$ (100 ml) and washed with water. The organic layer was concentrated under reduced pressure, yielding the following compounds, which were not purified but used as such for further reactions:

- 2-pivaloylamino-4-(tetrahydro-2H-pyran-4-yloxy)-6-(3,4-dimethoxyphenyl) pyrido[2,3d]pyrimidine (example 28) was obtained from tetrahydro-2H-pyran-4-ol; and
- 2-pivaloylamino-6-(3,4-dimethoxyphenyl)-4-(4-*N*-methylpiperidinoxy)pyrido [2,3-d]pyrimidine (example 29) was obtained from 1-methyl-4-piperidinol.

Examples 30 to 32 - synthesis of 2-amino-4-substituted-6-(3,4-dimethoxyphenyl) pyrido[2,3-d]pyrimidines

A mixture of a 2-pivaloylamino-4-substituted-6-(3,4-dimethoxyphenyl)pyrido [2,3-d]pyrimidine from examples 25-27 (1 mmole), $K_2CO_3$ (3 mmol) in MeOH (20 ml) and water (10 ml) was heated at 60 °C for 5 hours (with thin layer chromatography monitoring). The mixture was extracted with $CH_2Cl_2$ (100 ml). After concentration, the residue was purified by silica gel flash chromatography, the mobile phase being a $CH_3OH/CH_2Cl_2$ mixture, yielding the following compounds as yellowish solids which were characterized by their mass spectrum and their ultraviolet light spectrum as follows:

- 2-amino-4-*n*-propylamino-6-(3,4-dimethoxyphenyl)pyrido[2,3-d]pyrimidine (example 30) was obtained from the compound of example 25. UV (MeOH/$H_2O$, nm): 216.5, 268.4, 298.1, 357.6. MS (m/z): 310.1 ($[M+H]^+$, 100);
- 2-amino-4-isopropylamino-6-(3,4-dimethoxyphenyl)pyrido[2,3-d]pyrimidine (example 31) was obtained from the compound of example 26. UV (MeOH/$H_2O$, nm): 223.5, 268.4, 299.3, 357.6. MS (m/z): 310.2 ($[M+H]^+$, 100); and
- 2-amino-4-(1-piperidino)-6-(3,4-dimethoxyphenyl)pyrido[2,3-d]pyrimidine (example 32) was obtained from the compound of example 27. UV (MeOH/$H_2O$, nm): 217.6, 282.6, 373.1. MS (m/z): 366.3 ($[M+H]^+$, 100).

Examples 33 and 34 - synthesis of 2-amino-4-substituted-6-(3,4-dimethoxyphenyl) pyrido[2,3-d]pyrimidines

A solution of 2-pivaloylamino-4-substituted-6-(3,4-dimethoxyphenyl)pyrido [2,3-d]pyrimidine from example 28 or 29 (1 mmole), $K_2CO_3$ (3.0 mmol) in isopropanol (25 ml) and water (15 ml) was stirred at 70°C for 5 hours. The reaction mixture was extracted with dichloromethane. The organic layers were evaporated *in vacuo* and the crude residue was purified by silica gel column chromatography, the mobile phase being a 1:30 $CH_3OH/CH_2Cl_2$ mixture, yielding the following compounds as yellowish solids which were characterized by their mass spectrum and their ultraviolet light spectrum as follows:

- 2-amino-4-(tetrahydro-2H-pyran-4-yloxy)-6-(3,4-dimethoxyphenyl)pyrido[2,3-d]pyrimidine (example 33) was obtained from the compound of example 28. UV (MeOH/$H_2O$ nm): 222.3, 289.8, 362.5. MS (m/z): 383.1 ($[M+H]^+$, 100); and
- 2-amino-6-(3,4-dimethoxyphenyl)-4-(4-*N*-methylpiperidinoxy)pyrido[2,3-d]pyrimidine (example 34) was obtained from the compound of example 29. UV (MeOH/$H_2O$ nm): 222.3, 288.6, 362.5. MS (m/z): 813.1 ($[2M+Na]^+$, 30), 791.0 ($[2M+H]^+$, 30), 396.1 ($[M+H]^+$, 100).

Example 35 - synthesis of 2-amino-4-methoxy-6-(3,4-dimethoxyphenyl)pyrido[2,3d] pyrimidine

Sodium (92 mg, 4 mmole) was dissolved in methanol (20 ml) at room temperature, then 2-pivaloylamino-6-(3,4-dimethoxyphenyl)pyrido[2,3-d]pyrimidin-4-(3H)one (433 mg, 1 mmole) was added. The reaction mixture was stirred at room temperature for 30 minutes, and then refluxed for 2 hours. The reaction mixture was acidified with acetic acid (240 µl) and extracted with dichloromethane. The organic layers were evaporated *in vacuo* and the residue was purified by silica gel column chromatography, the mobile phase consisting of a 1:40 $CH_3OH/CH_2Cl_2$ mixture, resulting in the pure title compound as a white solid (257 mg, yield 82 %) which was characterized by its mass spectrum and its ultraviolet light spectrum as follows:

- UV (MeOH/$H_2O$, nm): 223.5, 289.8, 362.5; and
- MS (m/z): 313.1 ($[M+H]^+$, 100).

Example 36 - synthesis of 2-amino-4-(*sec*-butoxy)-6-(3,4-dimethoxyphenyl)-pyrido [2,3d]pyrimidine

Sodium (2 mmoles) was dissolved in isobutanol (30 ml) at 60 °C. Then, 2-pivaloylamino-4-(1,2,4-triazolyl)-6-(3,4-dimethoxyphenyl)pyrido[2,3-*d*]pyrimidine (1 mmole) from example 24 was added. The reaction mixture was stirred at 70 °C for 4 hours and then acidified with acetic acid (62.5 µl) and extracted with dichloromethane. The organic layers were evaporated *in vacuo* and the residue was purified by silica gel chromatography, the mobile phase consisting of a $CH_3OH/CH_2Cl_2$ mixture in a ratio of 1 :35 resulting in the title compound as a yellow solid (180 mg, yield 51 %) which was characterized by its mass spectrum and its ultraviolet light spectrum as follows:

- MS (m/z) : 355 ($[M+H]^+$, 100), and
- UV : 221, 289 and 362 nm.

Example 37 - synthesis of the hydrochloride salt of 2-amino-4-(*sec*-butoxy)-6-(3,4-dimethoxyphenyl)-pyrido[2,3-*d*]pyrimidine

The compound of example 36 (130 mg) was dissolved in MeOH. Then 0.36 ml of a 1.25 M HCl solution in methanol was added and the reaction mixture stirred at room temperature for 30 minutes. The solvents were evaporated *in vacuo,* resulting in the title compound as a pure yellow powder (130 mg, yield 90 %) which was characterized by its mass spectrum and its ultraviolet light spectrum as follows:

- MS (m/z) : 355 ($[M+H]^+$, 100), and
- UV : 221, 289 and 362 nm.

Example 38 - synthesis of 2-amino-4-isopropoxy-6-(3,4-dimethylphenyl)-pyrido[2,3-*d*]pyrimidine

A similar procedure as in example 2 was followed. To a solution of 2-pivaloylamino-4-isopropoxy-6-bromo-pyrido[2,3-d]pyrimidine (0.5 mmole) in tetrahydrofuran (15 ml) was added 3,4-dimethylphenyl boronic acid (0.5 mmole), tetrakis(triphenylphosphine)palladium(0) (0.025 mmole) and 15 ml of a 0.4 M $Na_2CO_3$ aqueous solution. The reaction mixture was refluxed at 95 °C for 30 minutes, yielding crude 2-pivaloylamino-4-isopropoxy-6-(3,4-dimethylphenyl)-pyrido [2,3-*d*]pyrimidine which was resuspended in a mixture of methanol (7 ml) and a 20 % $K_2CO_3$ aqueous solution (7 ml). The reaction mixture was refluxed for 2 hours, after which no pivaloylated material could be observed on thin layer chromatography. The solvents were evaporated *in vacuo* and the residue was purified by silica gel column chromatography, the mobile phase consisting of a $CH_3OH/CH_2Cl_2$ mixture in a ratio of 1:35, resulting in the title compound as a pure white powder (65 mg, yield 42 %) which was characterized by its mass spectrum and its ultraviolet light spectrum as follows:

- MS (m/z) : 309 ($[M+H]^+$, 100), and
- UV : 218, 242, 287 and 357 nm.

Examples 39 to 41 - synthesis of 2-pivaloylamino-4-substituted-6-(3,4-dimethoxyphenyl)pyrido[2,3-d]pyrimidines

While repeating the experimental procedure of examples 25-27, except for the starting amine, the following compounds were prepared :

- 2-pivaloylamino-4-(3-bromo-anilino)-6-(3,4-dimethoxyphenyl)pyrido[2,3-d]pyrimidine (example 39) was obtained from 3-bromo-aniline,
- 2-pivaloylamino-4-(benzylamino)-6-(3,4-dimethoxyphenyl)pyrido[2,3-d]pyrimidine (example 40) was obtained from benzylamine, and
- 2-pivaloylamino-4-(phenylethylamino)-6-(3,4-dimethoxyphenyl)pyrido[2,3-d]pyrimidine (example 41) was obtained from phenethylamine.

Examples 42 to 44 - synthesis of 2-amino-4-substituted-6-(3,4-dimethoxyphenyl) pyrido[2,3-d]pyrimidines

While repeating the experimental procedure of examples 30-32, but starting from the intermediates of examples 39-41, the following compounds were prepared and characterized by their mass spectrum and their ultraviolet light spectrum as follows:

- 2-amino-4-(3-bromo-anilino)-6-(3,4-dimethoxyphenyl)pyrido[2,3-d]pyrimidine (example 42): MS (m/z) : 451, 453 ($[M+H]^+$, 100); UV : 212, 261, 304 and 381 nm ;
- 2-amino-4-(benzylamino)-6-(3,4-dimethoxyphenyl)pyrido[2,3-d]pyrimidine (example 43): MS (m/z) : 387 ($[M+H]^+$, 100); UV : 212, 266, 303 and 366 nm ; and
- 2-amino-4-(phenylethylamino)-6-(3,4-dimethoxyphenyl)pyrido[2,3-d]pyrimidine (example 44) : MS (m/z) : 401 ($[M+H]^+$, 100) ; UV : 214, 267, 303 and 366 nm.

Example 45 - mixed lymphocyte reaction assay

Pyrido[2,3-d]pyrimidine derivatives were first dissolved (10mM) in dimethylsulfoxide (hereinafter referred as DMSO) and further diluted in culture medium before use for the following *in vitro* experiments. The commercially available culture medium consisted of RPMI-1640 + 10% foetal calf serum (FCS). Some pyrido[2,3-d]pyrimidine derivatives described herein were tested in the following mixed lymphocyte reaction (MLR) assay.

Peripheral blood mononuclear cells (hereinafter referred as PBMC) were isolated from heparinized peripheral blood by density gradient centrifugation over Lymphoprep (Nycomed, Maorstua, Norway). Allogeneic PBMC or Eppstein-Barr Virus-transformed human B cells [commercially available under the trade name RPMI1788 (ATCC name CCL156)] which strongly express B7-1 and B7-2 antigens were used as stimulator cells after irradiation with 30 Gy. MLR was performed in triplicate wells. After 5 days incubation at 37°C, 1 μCi [$^3$H]-thymidine was added to each cup. After a further 16 hours incubation, cells were harvested and counted in a ß-counter. Inhibition of proliferation by a compound described in some of the present examples was counted while using the formula:

$$\% \text{ inhibition} = \frac{(\text{cpm + drugs}) - (\text{cpm cult. med})}{(\text{cpm - drugs}) - (\text{OD cult. med})} \times 100$$

wherein cpm is the thymidine count per minute. The MLR assay is regarded by those skilled in the art as an *in vitro* analogue of the transplant rejection since it is based on the recognition of allogeneic major histocompatibility antigens on the stimulator leukocytes, by responding lymphocytes.

Table 1 below shows the $IC_{50}$ values for various pyrido[3,2-d]pyrimidines in the MLR test. The $IC_{50}$ value represents the lowest concentration of the pyrido[3,2-d]pyrimidine derivative (expressed in μmole/l) that resulted in a 50 % suppression of the MLR.

TABLE 1

| Example n° | MLR | Example n° | MLR | Example n° | MLR |
|---|---|---|---|---|---|
| 2 | 0.9 | 4 | 4.8 | 7 | 0.7 |
| 10 | 0.3 | 12 | 0.4 | 15 | 0.8 |
| 23 | 7.2 | 30 | 5.2 | 31 | 5.7 |
| 32 | 1.9 | 33 | 2.4 | 35 | 6.2 |
| 36 | 2.6 | 37 | 3.2 | 42 | 3.3 |
| 43 | 5.0 | 44 | 4.3 | | |

Example 46 - TNF-α assay

Peripheral blood mononuclear cells (herein referred as PBMC), in response to stimulation by lipopolysaccharide (hereinafter LPS), a gram-negative bacterial endotoxin, produce various chemokines, in particular human TNF-α. Inhibition of the activation of PBMC can therefore be measured by the level of suppression of the production of TNF-α by PBMC in response to stimulation by LPS.

Such inhibition measurement was performed as follows: PBMC were isolated from heparinized peripheral blood by density gradient centrifugation over Lymphoprep (commercially available from Nycomed, Norway). LPS was then added to the PMBC suspension in complete medium ($10^6$ cells /ml) at a final concentration of 1 μg/ml. The pyrido[3,2-d]pyrimidine derivative to be tested was added at different concentrations (0.1 μM, 1 pM and 10 μM) and the cells were incubated at 37°C for 72 hours in 5% $CO_2$. The supernatants were collected, then TNF-α concentrations were measured with respectively an anti-TNF-α antibody in a sandwich ELISA (Duo Set ELISA human TNFα, commercially available from R&D Systems, United Kingdom). The colorimetric reading of the ELISA was measured by a Multiskan RC plate reader (commercially available from ThermoLabsystems, Finland) at 450 nm (reference wavelength: 690 nm). Data analysis was performed with Ascent software 2.6. (also from ThermoLabsystems, Finland): a standard curve (recombinant human TNFα) was drawn and the amount (pg/ml) of each sample on the standard curve was determined.

The % suppression of human TNFα production by the pyrido[2,3-d]pyrimidine derivatives of the invention was calculated using the formula:

$$\% \text{ suppression} = \frac{\text{pg/ml in drugs} - \text{pg/ml in cult. med.}}{(\text{pg/ml in cult. med.+ LPS}) - \text{pg/ml cult. med.}}$$

Table 2 below shows the $IC_{50}$ values for various pyrido[3,2-d]pyrimidines in the TNFα assay.

TABLE 2

| Example n° | TNF α | Example n° | TNF α | Example n° | TNF α |
|---|---|---|---|---|---|
| 2 | 0.09 | 4 | 3.2 | 10 | 1.7 |
| 12 | 4.9 | 15 | 0.9 | 21 | 0.8 |
| 22 | 8.1 | 23 | 0.7 | 30 | 4.0 |
| 31 | 0.7 | 32 | 0.9 | 33 | 0.6 |
| 34 | 6.7 | 35 | 0.9 | 36 | 0.6 |
| 37 | 0.5 | 38 | 0.9 | 42 | 3.3 |
| 44 | 4.2 | | | | |

Example 47 - IL-1 B assay

Peripheral blood mononuclear cells (herein referred as PBMC), in response to stimulation by lipopolysaccharide (LPS), a gram-negative bacterial endotoxin, produce various chemokines, in particular human IL-1 β. Inhibition of the activation of PBMC can therefore be measured by the level of suppression of the production of IL-1 β by PBMC in response to stimulation by LPS.

Such inhibition measurement was performed as follows: PBMC were isolated from heparinized peripheral blood by density gradient centrifugation over Lymphoprep (commercially available from Nycomed, Norway). LPS was then added to the PMBC suspension in complete medium ($10^6$ cells /ml) at a final concentration of 1 μg/ml. The pyriso(2,3-d)pyrimidine derivative to be tested was added at different concentrations (0.1 μM, 1 μM and 10 μM) and the cells were incubated at 37°C for 72 hours in 5% $CO_2$. The supernatants were collected, then IL-1 β concentrations were measured with an anti-IL-1 β antibody in a sandwich ELISA. The colorimetric reading of the ELISA was measured by a Multiskan RC plate reader (commercially available from ThermoLabsystems, Finland) at 450 nm (reference wavelength: 690 nm). Data analysis was performed with Ascent software 2.6. (also from ThermoLabsystems, Finland) : a standard curve (recombinant human IL-1 β) was drawn and the amount (pg/ml) of each sample on the standard curve was determined.

The % suppression of human IL-1 β by the pyrido[2,3-d]pyrimidine derivatives of this invention was calculated using the formula:

$$\% \text{ suppression} = \frac{\text{pg/ml in drugs} - \text{pg/ml in cult. med.}}{(\text{pg/ml in cult. med.+ LPS}) - \text{pg/ml cult. med.}}$$

The $IC_{50}$ value represents the lowest concentration of the pyrido[3,2-d]pyrimidine derivative (expressed in μmole/l) that resulted in a 50 % suppression of human IL-1 β. The following $IC_{50}$ values in the IL-1β test were obtained:

Compound of example 15: 7.0 μmole/l
Compound of example 42: 7.0 μmole/l

Example 48 - synthesis of 2-pivaloylamino-4-*N*-homopiperazino-6-bromo-pyrido[2,3-d]pyrimidine and 2-amino-4-*N*-homopiperazino-6-bromo-pyrido[2,3-d]pyrimidine

2-pivaloylamino-4-*N*-homopiperazino-6-bromo-pyrido[2,3-d]pyrimidine is prepared in good yield by analogy with the synthesis procedure described in example 5, but starting from homopiperazine instead of piperazine. The pivaloyl protecting group in position 2 of the pyrido[2,3-d]pyrimidine ring may then be conveniently removed by acidic hydrolysis.

Example 49 - synthesis of 2-pivaloylamino-4-*N*-homopiperazino-6-(3,4-dimethoxyphenyl)-pyrido[2,3-d]pyrimidine and 2-amino-4-*N*-homopiperazino-6-(3,4-dimethoxyphenyl)-pyrido[2,3-d]pyrimidine

2-pivaloylamino-4-*N*-homopiperazino-6-(3,4-dimethoxy-phenyl)-pyrido[2,3-d]pyrimidine is prepared in good yield by analogy with the synthesis procedure described in example 8, but starting from the compound of example 48.

The pivaloyl protecting group in position 2 of the pyrido[2,3-d]pyrimidine ring may then be conveniently removed by acidic hydrolysis.

Example 50 - synthesis of 2-pivaloylamino-6-(4-fluorophenyl)pyrido[2,3-d]pyrimidin-4-(3H)-one

To a solution of 2-pivaloylamino-6-bromo-pyrido[2,3-d]pyrimidin-4-(3H)one (975 mg, 3 mmole), synthesized and characterized according to Taylor et al. in Hetercycles (1993) 36:1883, in 1,4-dioxane (90 ml) and water (36 ml) was added 4-fluorophenyl boronic acid (462 mg, 3.3 mmole), $K_2CO_3$ (1.04 g, 7.5 mmole) and tetrakis(triphenylphosphine) palladium(0) (173 mg, 0.15 mmole). The reaction mixture was heated for 2 hours at 90°C. The reaction mixture was extracted with dichloromethane and the organic phase was concentrated under reduced pressure. The residue was purified by silica gel flash chromatography, the mobile phase being a 1:99 MeOH/$CH_2Cl_2$ mixture, resulting in 2-pivaloylamino-6-(4-fluorophenyl) pyrido[2,3-d]pyrimidin-4-(3H)-one (384 mg, yield : 38 %), characterized by its mass spectrum as follows : MS (m/z) : 341 ($[M+H]^+$, 100).

Example 51 - synthesis of 2-pivaloylamino-4-(1,2,4-triazolyl)-6-(4-fluorophenyl)pyrido [2,3-d]pyrimidine

To a solution of 2-pivaloylamino-6-(4-fluorophenyl)pyrido[2,3-d]pyrimidin-4-(3H)-one (600 mg, 1.8 mmole) in acetonitrile (45 ml) were added 1,2,4-triazole (1.218 g, 18 mmole), triethylamine (535 mg, 5.3 mmole) and $POCl_3$ (493 μl, 5.3 mmole). The reaction mixture was stirred at room temperature for 24 hours (with thin layer chromatography monitoring). The reaction mixture was diluted with dichloromethane (70 ml) and washed with a 2% aqueous hydrochloric acid solution. The organic layer was concentrated under reduced pressure yielding crude 2-pivaloylamino-4-(1,2,4-triazolyl)-6-(4-fluorophenyl)pyrido[2,3-d]pyrimidine, which was used for further reaction without any purification.

Example 52 - synthesis of 2-pivaloylamino-4-(3-methylanilino)-6-bromo-pyrido]2,3-d]pyrimidine

To a suspension of 2-pivaloylamino-4-(1,2,4-triazolyl)-6-bromo-pyrido[2,3-d] pyrimidine (605 mg, 1.6 mmol), for instance prepared and characterized according to Taylor et al. Heterocycles (1993) 36:1889, in 1,4-dioxane (30 ml) was added *m*-toluidine (172 mg, 1.6 mmole). The suspension became a yellow solution after 1 hour and was further stirred at room temperature for 16 hours. Water was added to the reaction mixture, which was then extracted three times with dichloromethane. The organic layers were evaporated under reduced pressure and the crude residue was purified by silica gel flash chromatography, the mobile phase being a 1:99 MeOH/$CH_2Cl_2$ mixture, resulting in the pure title compound as a yellowish powder (346 mg, yield : 52%) which was characterized by its mass spectrum as follows : MS (m/z) : 415 ($[M+H]^+$, 100).

Example 53 - synthesis of 2-amino-4-(3-methylanilino)-6-(4-fluorophenyl)pyrido[2,3-d]pyrimidine

To a solution of 2-pivaloylamino-4-(3-methylanilino)-6-bromo-pyrido[2,3-d]pyrimidine (297 mg, 0.72 mmole) in 1,4-dioxane (25 ml) and water (10 ml) was added 4-fluorophenyl boronic acid (110 mg, 0.79 mmole), $K_2CO_3$ (248 mg, 1.8 mmole) and tetrakis(triphenylphosphine)palladium(0) (0.03 mmole, 41 mg). The reaction mixture was heated to 90°C for 17 hours. The reaction mixture was concentrated under reduced pressure and the residue was dissolved in methanol (20 ml). To this solution was added an aqueous solution of $K_2CO_3$ (20%, 10 ml). The reaction mixture was heated at 60°C for 2 hours. The reaction mixture was then extracted with dichloromethane and the organic phase was concentrated under reduced pressure. The residue was purified by silica gel flash chromatography, the mobile phase being a 1:30 MeOH/$CH_2Cl_2$ mixture resulting in the title compound as a yellowish solid (112 mg, yield : 45%) which was characterized by its mass spectrum as follows : MS (m/z) : 346 ($[M+H]^+$, 100).

Examples 54 to 57 - synthesis of 2-pivaloylamino-4-substituted-6-(4-fluorophenyl) pyrido[2,3-d]pyrimidine analogues

The crude 2-pivaloylamino-4-(1,2,4-triazolyl)-6-(4-fluorophenyl)pyrido[2,3-d]pyrimidine from example (0.6 mmole) was dissolved in 1,4-dioxane (15 ml), and an appropriate amine (3 equivalents) was added. The mixture was stirred at room temperature for 12 hours. The solvents were evaporated under reduced pressure yielding the following crude compounds, which were used for further reaction without any purification:

- 2-pivaloylamino-4-(*N*-piperidino)-6-(4-fluorophenyl)pyrido[2,3-d]pyrimidine (example 54) was obtained from piperidine;
- 2-pivaloylamino-4-(*N*-morpholino)-6-(4-fluorophenyl)pyrido[2,3-d]pyrimidine (example 55) was obtained from morpholine;
- 2-pivaloylamino-4-(*N*-acetyl-*N*-piperazin-1-yl)-6-(4-fluorophenyl)pyrido[2,3-d]pyrimidine (example 56) was obtained

from *N*-acetyl piperazine; and

- 2-pivaloylamino-4-(*N*-methyl-*N*-piperazin-1-yl)-6-(4-fluorophenyl)pyrido[2,3-d]pyrimidine (example 57) was obtained from *N*-methyl piperazine.

Examples 58 to 61 - synthesis of 2-amino-4-substituted-6-(4-fluorophenyl)pyrido[2,3-d]pyrimidine analogues

A mixture of a 2-pivaloylamino-4-substituted-6-(4-fluorophenyl)pyrido[2,3-d]pyrimidine from examples 53-57 (0.5 mmole), $K_2CO_3$ (1.5 mmole) in methanol (10 ml) and water (6 ml) was heated at 50°C (with thin layer chromatography monitoring). The mixture was extracted with dichloromethane. After concentration, the residue was purified by silica gel flash chromatography, the mobile phase being a $MeOH/CH_2Cl_2$ mixture, yielding the following compounds as yellowish solids which were characterized by their mass spectrum as follows:

- 2-amino-4-(*N*-piperidino)-6-(4-fluorophenyl)pyrido[2,3-d]pyrimidines (example 58) was obtained from the compound of example 54 after 24 hours of heating. (102 mg, yield: 63%), MS (m/z): ($[M+H]^+$, 100)
- 2-amino-4-(*N*-morpholino)-6-(4-fluorophenyl)pyrido[2,3-d]pyrimidines (example 59) was obtained from the compound of example 55 after 5 hours of heating. (73 mg, yield: 45%), MS (m/z): ($[M+H]^+$ 100)
- 2-amino-4-(*N*-acetyl-*N*-piperazin-1-yl)-6-(4-fluorophenyl)pyrido[2,3-d]pyrimidines (example 60) was obtained from the compound of example 56 after 5 hours of heating. (108 mg, yield: 59%), MS (m/z) : ($[M+H]^+$, 100)
- 2-amino-4-(*N*-methyl-*N*-piperazin-1-yl)-6-(4-fluorophenyl)pyrido[2,3-d]pyrimidines (example 61) was obtained from the compound of example 57 after 8 hours of heating. (51 mg, yield: 30%), MS (m/z) : ($[M+H]^+$, 100).

Examples 62 and 63 - synthesis of 2-pivaloylamino-4-alkoxy-6-(4-fluorophenyl) pyrido [2,3-d]pyrimidines

Sodium (3.5 mmole) was dissolved in an appropriate alcohol (7 mmole) and tetrahydrofuran (10 ml) by refluxing. After cooling to room temperature, the crude 2-pivaloylamino-4-(1,2,4-triazolyl)-6-(4-fluorophenyl)pyrido[2,3-d]pyrimidine from example (0.7 mmole) was added as a suspension in tetrahydrofuran (10 ml). The mixture was stirred at room temperature for 1 hour. The mixture was diluted with dichloromethane (100 ml) and washed with water. The organic layer was concentrated under reduced pressure, yielding the following compounds, which were not purified but used as such for further reactions:

- 2-pivaloylamino-4-ethoxy-6-(4-fluorophenyl)pyrido[2,3-d]pyrimidine, (example 62) was obtained from ethanol;
- 2-pivaloylamino-4-isopropoxy-6-(4-fluorophenyl)pyrido[2,3-d]pyrimidine (example 63) was obtained from isopropanol.

Example 64 - synthesis of 2-amino-4-ethoxy-6-(4-fluorophenyl)pyrido[2,3-d]pyrimidine

A mixture of 2-pivaloylamino-4-ethoxy-6-(4-fluorophenyl)pyrido[2,3-d]pyrimidine from example 62 (0.44 mmole), $K_2CO_3$ (1.3 mmole) in ethanol (10 ml) and water (6 ml) was heated at 70°C (with thin layer chromatography monitoring) for 4 hours. The mixture was extracted with dichloromethane. After concentration, the residue was purified by silica gel flash chromatography, the mobile phase being a 1:30 $MeOH/CH_2Cl_2$ mixture, yielding the pure title compound as a yellowish solid (57 mg, yield : 46%) which was characterized by its mass spectrum as follows : MS (m/z) : 285 ($[M+H]^+$, 100).

Example 65 - synthesis of 2-amino-4-isopropoxy-6-(4-fluorophenyl)pyrido[2,3-d]pyrimidine

A mixture of 2-pivaloylamino-4-isopropoxy-6-(4-fluorophenyl)pyrido[2,3-d]pyrimidine from example 63 (0.52 mmole), $K_2CO_3$ (1.5 mmole) in isopropanol (15 ml) and water (9 ml) was heated at 70°C (with thin layer chromatography monitoring). The mixture was extracted with dichloromethane. The combined organic layers were evaporated, and after concentration under reduced pressure, the residue was purified by silica gel flash chromatography, the mobile phase being a 2:98 $MeOH/CH_2Cl_2$ mixture, yielding the title compound as a yellowish solid (47 mg, yield : 30%) which was characterized by its mass spectrum as follows : MS (m/z) : 299 ($[M+H]^+$, 100).

## Claims

**1.** A pyrido(2,3-d)pyrimidine derivative having the general formula:

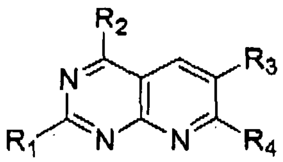

wherein:

- $R_1$ is amino,
- $R_2$ is selected from the group consisting of hydroxy, halogen, mono $C_{1-7}$alkyl amino; mono-arylamino; mono-aryl$C_{1-7}$alkylamino; morpholinyl; N-piperidinyl; triazolyl; heterocyclic-substituted amino; $C_{1-7}$ alkoxy; oxyheterocyclic; piperazinyl or homopiperazinyl, wherein said piperazinyl is optionally N-substituted with acyl $C_{1-7}$ alkyl or arylalkyl;
- $R_3$ is selected from the group consisting of aryl and heteroaryl groups optionally substituted with one or more substituents selected from the group consisting of halogen, halo $C_{1-7}$ alkyl, $C_{1-7}$ alkoxy, and alkylamino, and
- $R_4$ is hydrogen,

or a pharmaceutical acceptable addition salt thereof, or a dihydro-derivative or a tetrahydro-derivative thereof, or a stereochemical isomeric form thereof, or a N-oxide thereof, or a solvate thereof.

**2.** A pyrido(2,3-d)pyrimidine derivative according to claim 1, wherein $R_2$ is halogen or triazolyl.

**3.** A pyrido(2,3-d)pyrimidine derivative according to any of claims 1 to 4, wherein $R_3$ is a monosubstituted or polysubstituted phenyl group.

**4.** A pyrido(2,3-d)pyrimidine derivative according to any of claims 1, 2 and 4 to 8, wherein $R_3$ is a phenyl group having no more than two substituents and wherein one substituent is in a para position on said phenyl ring.

**5.** A pyrido(2,3-d)pyrimidine derivative according to any of claims 1 to 4, wherein $R_3$ is thienyl.

**6.** A pyrido(2,3-d)pyrimidine derivative according to any of claims 1 and 3 to 5, wherein $R_2$ is tetrahydro-pyranyloxy or methylpiperidinoxy.

**7.** A pyrido(2,3-d)pyrimidine derivative according to any of claims 1 and 3 to 5, wherein $R_2$ is $C_{1-4}$ alkoxy.

**8.** A pyrido(2,3-d)pyrimidine derivative according to any of claims 1 and 3 to 5, wherein $R_2$ is monopropylamino, anilino, bromoanilino, benzylamino or phenylethylamino.

**9.** A pyrido(2,3-d)pyrimidine derivative according to any of claims 1 and 3 to 5, wherein $R_2$ is a piperazin-1-yl group, said group being optionally substituted in the 4 position with a substituent $R_5$, wherein $R_5$ is selected from the group consisting of:

- $COR_8$ wherein $R_8$ is selected from hydrogen, $C_{1-7}$ alkyl, aryl optionally substituted with one or more substituents, aryloxy, and arylamino; and
- $R_{11}$, wherein $R_{11}$ is arylalkyl.

**10.** A pyrido(2,3-d)pyrimidine derivative according to any of claims 1 and 3 to 5, wherein $R_2$ is methylphenylcarbamoyl-piperazin-1-yl, *N*-phenoxyacetyl-*N*-piperazin-1-yl, (*N*-benzyloxycarbonyl)piperazin-1-yl or *N*-benzylpiperazin-1-yl,

**11.** A pyrido(2,3-d)pyrimidine derivative, being selected from the group consisting of:

- 2-amino-4-isopropoxy-6-bromo-pyrido[2,3-d]pyrimidine.
- 2-amino-4-isopropoxy-6-(3,4-dimethoxyphenyl)-pyrido[2,3-d]pyrimidine, 2-pivaloylamino-4-morpholino-6-bromo-pyrido[2,3-d]pyrimidine,
- 2-amino-4-morpholino-6-(3,4-dimethoxyphenyl)-pyrido[2,3-d]pyrimidine,
- 2-pivaloylamino-4-*N*-piperazino-6-bromo-pyrido[2,3-d]pyrimidine.
- 2-pivaloylamino-4-[N-4-methylphenylcarbamoyl-piperazin-1-yl]-6-bromo-pyrido[2,3-d]pyrimidine,

- 2-amino-4-[N-4-methylphenylcarbamoyl-piperazin-1-yl]-6-bromo-pyrido[2,3-d]pyrimidine,
- 2-pivaloylamino-4-*N*-piperazinyl-6-(3,4-dimethoxyphenyl)-pyrido [2,3-d]pyrimidine,
- 2-pivaloylamino-4-[(N-phenoxyacetyl)-N-piperazin-1-yl]-6-(3,4-dimethoxyphenyl)-pyrido[2,3-d]pyrimidine.
- 2-amino-4-(*N*-phenoxyacetyl-*N*-piperazin-1-yl)-6-(3,4-dimethoxyphenyl)pyrido[2, 3d]pyrimidine,
- 2-pivaloylamino-4-[N-4-methylphenylcarbamoyl-piperazin-1-yl]-6-(3,4-dimethoxyphenyl)pyrido[2,3-d]pyrimidine,
- 2-amino-4-(*N*-4-methylphenylcarbamoyl-piperazin-1-yl]-6-(3,4-dimethoxyphenyl) pyrido[2,3-d]pyrimidine,
- 2-pivaloylamino-4-[(N-benzyloxycarbonyl)-piperazin-1-yl]-6-bromo-pyrido[2,3-d]pyrimidine,
- 2-pivaloylamino-4-[(*N*-benzyloxycarbonyl)-piperazin-1-yl]-6-(3,4-dimethoxyphenyl) -pyrido[2,3-d]pyrimidine,
- 2-amino-6-(3,4-dimethoxyphenyl)-4-[(*N*-benzyloxycarbonyl)-piperazin-1-yl]-pyrido [2,3-d]pyrimidine,
- 2-pivaloylamino-4-isopropoxy-6-(4-dimethylaminophenyl)pyrido[2,3-d]pyrimidine,
- 2-pivaloylamino-4-isopropoxy-6-(4-trifluoromethylphenyl)pyrido[2,3-d]pyrimidine;
- 2-pivaloylamino-4-isopropoxy-6-(2-thienyl)pyrido[2,3-d]pyrimidine,
- 2-amino-4-isopropoxy-6-(4-dimethylaminophenyl)pyrido[2,3-d]pyrimidine;
- 2-amino-4-isopropoxy-6-(4-trifluoromethylphenyl)-pyrido[2,3-d]pyrimidine,
- 2-amino-4-isopropoxy-6-(2-thienyl)pyrido[2,3-d]pyrimidine,
- 2-amino-4-(piperazin-1-yl)-6-(3,4-dimethoxyphenyl)-pyrido[2,3-d]pyrimidine,
- 2-amino-4-(4-benzylpiperazin-1-yl)-6-(3,4-dimethoxyphenyl)-pyrido[2,3-d]pyrimidine,
- 2-pivaloylamino-4-(1,2,4-triazolyl)-6-(3,4-dimethoxyphenyl)pyrido[2,3-d]pyrimidine,
- 2-pivaloylamino-4-n-propylamino-6-(3,4-dimethoxyphenyl)pyrido[2,3-d]pyrimidine;
- 2-pivaloylamino-4-isopropylamino-6-(3,4-dimethoxyphenyl)pyrido[2,3-d]pyrimidine;
- 2-pivaloylamino-4-(1-piperidino)-6-(3,4-dimethoxyphenyl)pyrido[2,3-d]pyrimidine;
- 2-pivaloylamino-4-(tetrahydro-2H-pyran-4-yloxy)-6-(3,4-dimethoxyphenyl)pyrido [2,3-d]pyrimidine,
- 2-pivaloylamino-6-(3,4-dimethoxyphenyl)-4-(4-*N*-methylpiperidinoxy)pyrido[2,3-d]pyrimidine;
- 2-amino-4-*n*-propylamino-6-(3,4-dimethoxyphenyl)pyrido[2,3-d]pyrimidine;
- 2-amino-4-isopropylamino-6-(3,4-dimethoxyphenyl)pyrido[2,3-d]pyrimidine;
- 2-amino-4-(1-piperidino)-6-(3,4-dimethoxyphenyl)pyrido[2,3-d]pyrimidine;
- 2-amino-4-(tetrahydro-2H-pyran-4-yloxy)-6-(3,4-dimethoxyphenyl)pyrido[2,3-d] pyrimidine;
- 2-amino-6-(3,4-dimethoxyphenyl)-4-(4-*N*-methylpiperidinoxy)pyrido[2,3-d]pyrimidine;
- 2-amino-4-methoxy-6-(3,4-dimethoxyphenyl)pyrido[2,3d]pyrimidine;
- 2-pivaloylamino-4-*N*-homopiperazino-6-bromo-pyrido[2,3-d]pyrimidine;
- 2-pivaloylamino-4-*N*-homopiperazino-6-(3,4-dimethoxy-phenyl)-pyrido[2,3-d]pyrimidine;
- 2-amino-4-*N*-homopiperazino-6-bromo-pyrido[2,3-d]pyrimidine;
- 2-amino-4-*N*-homopiperazino-6-(3,4-dimethoxyphenyl)-pyrido[2,3-d]pyrimidine:
- 2-amino-4-(*sec*-butoxy)-6-(3,4-dimethoxyphenyl)-pyrido [2,3d]pyrimidine;
- 2-amino-4-(*sec*-butoxy)-6-(3,4-dimethoxyphenyl)-pyrido[2,3d]pyrimidine hydrochloride salt;
- 2-amino-4-isopropoxy-6-(3,4-dimethy)phenyl)-pyrido[2,3-*d*]pyrimidine;
- 2-pivaloylamino-4-(3-bromo-anilino)-6-(3,4-dimethoxyphenyl)pyrido[2,3-d]pyrimidine ;
- 2-pivaloylamino-4-(benzylamino)-6-(3,4-dimethoxyphenyl)pyrido[2,3-d]pyrimidine ;
- 2-pivaloylamino-4-(phenylethylamino)-6-(3,4-dimethoxyphenyl)pyrido[2,3-d]pyrimidine ;
- 2-amino-4-(3-bromo-anilino)-6-(3,4-dimethoxyphenyl)pyrido[2,3-d]pyrimidine;
- 2-amino-4-(benzylamino)-6-(3,4-dimethoxyphenyl)pyrido[2,3-d]pyrimidine;
- 2-amino-4-(phenylethylamino)-6-(3,4-dimethoxyphenyl)pyrido[2,3-d]pyrimidine;
- 2-pivaloylamino-4-*N*-homopiperazino-6-bromo-pyrido[2,3-d]pyrimidine;
- 2-amino-4-*N*-homopiperazino-6-bromo-pyrido[2,3-d]pyrimidine;
- 2-pivaloylamino-4-*N*-homopiperazino-6-(3,4-dimethoxy-phenyl)-pyrido[2,3-d]pyrimidine;
- 2-amino-4-*N*-homopiperazino-6-(3,4-dimethoxy-phenyl)-pyrido[2,3-d]pyrimidine;
- 2-pivaloylamino-6-(4-fluorophenyl) pyrido[2,3-d]pyrimidin-4-(3H)-one;
- 2-pivaloylamino-4-(1,2,4-triazolyl)-6-bromo-pyrido[2,3-d] pyrimidine:
- 2-pivaloylamino-4-(3-methylanilino)-6-bromo-pyrido[2,3-d]pyrimidine;
- 2-amino-4-(3-methylanilino)-6-bromo-pyrido[2,3-d]pyrimidine;
- 2-pivaloylamino-4-(*N*-piperidino)-6-(4-fluorophenyl)pyrido[2,3-d]pyrimidine;
- 2-pivaloylamino-4-(*N*-morpholino)-6-(4-fluorophenyl)pyrido[2,3-d]pyrimidine;
- 2-pivaloylamino-4-(*N*-acetyl-*N*-piperazin-1-yl)-6-(4-fluorophenyl)pyrido[2,3-d]pyrimidine;
- 2-pivaloylamino-4-(*N*-methyl-*N*-piperazin-1-yl)-6-(4-fluorophenyl)pyrido[2,3-d]pyrimidine;
- 2-amino-4-(*N*-piperidino)-6-(4-fluorophenyl)pyrido[2,3-d]pyrimidine;
- 2-amino-4-(*N*-morpholino)-6-(4-fluorophenyl)pyrido[2,3-d]pyrimidine;
- 2-amino-4-(*N*-acetyl-*N*-piperazin-1-yl)-6-(4-fluorophenyl)pyrido[2,3-d]pyrimidine;

- 2-amino-4-(*N*-methyl-*N*-piperazin-1-yl)-6-(4-fluorophenyl)pyrido[2,3-d]pyrimidine;
- 2-pivaloylamino-4-ethoxy-6-(4-fluorophenyl)pyrido[2,3-d]pyrimidine;
- 2-pivaloylamino-4-isopropoxy-6-(4-fluorophenyl)pyrido[2,3-d]pyrimidine;
- 2-amino-4-ethoxy-6-(4-fluorophenyl)pyrido[2,3-d]pyrimidine; and
- 2-amino-4-isopropoxy-6-(4-fluorophenyl)pyrido[2,3-d]pyrimidine.

**12.** A pharmaceutical composition comprising a pyrido(2,3-d)pyrimidine derivative according to claim 1.

**13.** A pharmaceutical composition according to claim 12, wherein $R_3$ is a monosubstituted or polysubstituted phenyl group.

**14.** A pharmaceutical composition according to claim 12 or claim 13, wherein $R_3$ is a phenyl group having no more than two substituents and wherein one substituent is in a para position on said phenyl ring.

**15.** A pharmaceutical composition according to claim 12, wherein $R_3$ is thienyl.

**16.** A pharmaceutical composition according to any of claims 12 to 15, further comprising one or more pharmaceutically acceptable carriers.

**17.** A pharmaceutical composition according to any of claims 12 to 16, further comprising one or more biologically-active drugs being selected from the group consisting of immunosuppressant and/or immunomodulator drugs, and antineoplastic drugs.

**18.** A process for making a pyrido(2,3-d)pyrimidine derivative according to any of claims 1 to 11, comprising a step of reacting a 2-$R_1$-substituted-6-bromo-pyrido[2,3-d]pyrimidine or a 2-$R_1$-substituted-6-chloro-pyrido[2,3-d]pyrimidine, wherein $R_1$ is as defined in the general formula (I), with an aryl boronic acid including the radical $R_3$ wherein $R_3$ is an aryl or heteroaryl group as defined in the general formula (I).

**19.** A process according to claim 18. wherein $R_1$ is an amino group, and wherein said process includes a protection step for said amino group.

**20.** A process according to claim 18 or claim 19, further comprising a step of activating a hydroxyl group at position 4 of the pyrido[2,3-d]pyrimidine scaffold.

**21.** A pyrido(2,3-d)pyrimidine derivative according to any of claims 1 to 11 for use in prevention or treatment of immune disorders, autoimmune disorders, cardiovascular disorders, disorders of the central nervous system, TNF-alpha related disorders and cell proliferative disorders.

## Patentansprüche

**1.** Pyrido(2,3-d)pyrimidinderivat mit der allgemeinen Formel

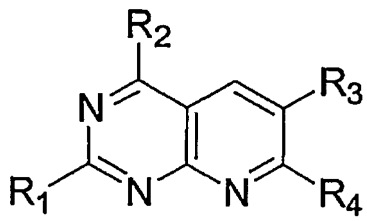

wobei:

- $R_1$ Amino ist
- $R_2$ ausgewählt ist aus der Gruppe bestehend aus Hydroxy, Halogen, Mono-$C_{1-7}$-alkylamino; Monoarylamino; Monoaryl-$C_{1-7}$-alkylamino; Morpholinyl; N-Piperidinyl; Triazolyl; heterocyclisch substituiertem Amino; $C_{1-7}$-alk-

oxy-, Oxyheterocyclyl; Piperazinyl oder Homopiperazinyl, wobei das Piperazinyl gegebenenfalls mit Acyl-$C_{1-7}$-alkyl oder Arylalkyl N-substituiert ist
- $R_3$ ausgewählt ist aus der Gruppe bestehend aus Aryl- und Heteroarylgruppen, die gegebenenfalls substituiert sind mit einem oder mehreren Substituenten ausgewählt aus der Gruppe bestehend aus Halogen, Halogen-$C_{1-7}$-alkyl, $C_{1-7}$-alkoxy und Alkylamino, und
- $R_4$ Wasserstoff ist

oder ein pharmazeutisch akzeptables Additionssalz davon oder ein Dihydro-Derivat oder ein Tetrahydro-Derivat davon oder eine stereochemisch isomere Form davon oder ein N-Oxid davon oder ein Solvat davon.

**2.** Pyrido(2,3-d)pyrimidinderivat gemäß Anspruch 1, wobei $R_2$ Halogen oder Triazolyl ist.

**3.** Pyrido(2,3-d)pyrimidinderivat gemäß einem der Ansprüche 1 bis 4, wobei $R_3$ eine monosubstituierte oder polysubstituierte Phenylgruppe ist.

**4.** Pyrido(2,3-d)pyrimidinderivat gemäß einem der Ansprüche 1, 2 und 4 bis 8, wobei $R_3$ eine Phenylgruppe ist, die nicht mehr als zwei Substituenten hat und wobei ein Substituent paraständig am Phenylring ist.

**5.** Pyrido(2,3-d)pyrimidinderivat gemäß einem der Ansprüche 1 bis 4, wobei $R_3$ Thienyl ist.

**6.** Pyrido(2,3-d)pyrimidinderivat gemäß einem der Ansprüche 1 und 3 bis 5, wobei $R_2$ Tetrahydropyranyloxy oder Methylpiperidinoxy ist.

**7.** Pyrido(2,3-d)pyrimidinderivat gemäß einem der Ansprüche 1 und 3 bis 5, wobei $R_2$ $C_{1-4}$-alkoxy ist.

**8.** Pyrido(2,3-d)pyrimidinderivat gemäß einem der Ansprüche 1 und 3 bis 5, wobei $R_2$ Monopropylamino, Anilino, Bromanilino, Benzylamino oder Phenylethylamino ist.

**9.** Pyrido(2,3-d)pyrimidinderivat gemäß einem der Ansprüche 1 und 3 bis 5, wobei $R_2$ eine Piperazin-1-yl-gruppe ist, wobei die Gruppe gegebenenfalls in der 4-Position mit einem Substituenten $R_5$ substituiert ist, wobei $R_5$ ausgewählt ist aus der Gruppe bestehend aus:

- $COR_8$, wobei $R_8$ ausgewählt ist aus Wasserstoff, $C_{1-7}$-alkyl, Aryl, das gegebenenfalls mit einem oder mehreren Substituenten substituiert ist, Aryloxy und Arylamino; und
- $R_{11}$, wobei $R_{11}$ Arylalkyl ist.

**10.** Pyrido(2,3-d)pyrimidinderivat gemäß einem der Ansprüche 1 und 3 bis 5, wobei $R_2$ Methylphenylcarbamoylpiperazin-1-yl, N-Phenoxyacetyl-N-piperazin-1-yl,(N-Benzyloxycarbonyl)piperazin-1-yl oder N-benzylpiperazin-1-yl ist.

**11.** Pyrido(2,3-d)pyrimidinderivat, das ausgewählt ist aus der Gruppe bestehend aus:

- 2-Amino-4-isopropoxy-6-brom-pyrido[2,3-d]pyrimidin,
- 2-Amino-4-isopropoxy-6-(3,4-dimethoxyphenyl)-pyrido[2,3-d]pyrimidin,
- 2-Pivaloylamino-4-morpholino-6-brom-pyrido[2,3-d]pyrinnidin,
- 2-Amino-4-morpholino-6-(3,4-dimethoxyphenyl)-pyrido[2,3-d]pyrimidin,
- 2-Pivaloylamino-4-*N*-piperazino-6-brom-pyrido[2,3-d]pyrimidin,
- 2-Pivaloylamino-4-[*N*-4-methylphenylcarbamoyl-piperazin-1-yl]-6-brom-pyrido[2,3-d]pyrimidin,
- 2-Amino-4-[*N*-4-methylphenylcarbamoyl-piperazin-1-yl]-6-brom-pyrido[2,3-d]pyrimidin,
- 2-Pivaloylamino-4-*N*-piperazinyl-6-(3,4-dimethoxyphenyl)-pyrido[2,3-d]pyrimidin,
- 2-Pivaloylamino-4-[(*N*-phenoxyacetyl)-*N*-piperazin-1-yl]-6-(3,4-dimethoxyphenyl)-pyrido[2,3-d]pyrimidin,
- 2-Amino-4-(*N*-phenoxyacetyl-*N*-piperazin-1-yl)-6-(3,4-dimethoxyphenyl)-pyrido[2,3-d]pyrimidin,
- 2-Pivaloylamino-4-[*N*-4-methylphenylcarbamoyl-piperazin-1-yl]-6-(3,4-dimethoxy-phenyl)-pyrido[2,3-d]pyrimidin,
- 2-Amino-4-(*N*-4-methylphenylcarbamoyl-piperazin-1-yl]-6-(3,4-dimethoxyphenyl)-pyrido[2,3-d]pyrimidin,
- 2-Pivaloylamino-4-[(*N*-benzyloxycarbonyl)-piperazin-1-yl]-6-brom-pyrido[2,3-d]pyrimidin,
- 2-Pivaloylamino-4-[(*N*-benzyloxycarbonyl)-piperazin-1-yl]-6-(3,4-dimethoxyphenyl)-pyrido[2,3-d]pyrimidin,
- 2-Amino-6-(3,4-dimethoxyphenyl)-4-[(*N*-benzyloxycarbonyl)-piperazin-1-yl]-pyrido[2,3-d]pyrimidin,
- 2-Pivaloylamino-4-isopropoxy-6-(4-dimethylaminophenyl)-pyrido[2,3-d]pyrimidin,

- 2-Pivaloylamino-4-isopropoxy-6-(4-trifluormethylphenyl)-pyrido[2,3-d]pyrimidin;
- 2-Pivaloylamino-4-isopropoxy-6-(2-thienyl)-pyrido[2,3-d]pyrimidin,
- 2-Amino-4-isopropoxy-6-(4-dimethylaminophenyl)-pyrido[2,3-d]pyrimidin;
- 2-Amino-4-isopropoxy-6-(4-trifluormethylphenyl)-pyrido[2,3-d]pyrimidin,
- 2-Amino-4-isopropoxy-6-(2-thienyl)-pyrido[2,3-d]pyrimidin,
- 2-Amino-4-(piperazin-1-yl)-6-(3,4-dimethoxyphenyl)-pyrido[2,3-d]pyrimidin,
- 2-Amino-4-(4-benzylpiperazin-1-yl)-6-(3,4-dimethoxyphenyl)-pyrido[2,3-d]pyrimidin,
- 2-Pivaloylamino-4-(1,2,4-triazolyl)-6-(3,4-dimethoxyphenyl)-pyrido[2,3-d]pyrimidin,
- 2-Pivaloylamino-4-*n*-propylamino-6-(3,4-dimethoxyphenyl)-pyrido[2,3-d]pyrimidin;
- 2-Pivaloylamino-4-isopropylamino-6-(3,4-dimethoxyphenyl)-pyrido[2,3-d]pyrimidin;
- 2-Pivaloylamino-4-(1-piperidino)-6-(3,4-dimethoxyphenyl)-pyrido[2,3-d]pyrimidin;
- 2-Pivaloylamino-4-(tetrahydro-2H-pyran-4-yloxy)-6-(3,4-dimethoxyphenyl)-pyrido[2,3-d]pyrimidin,
- 2-Pivaloylamino-6-(3,4-dimethoxyphenyl)-4-(4-*N*-methylpiperidinoxy)-pyrido[2,3-d]pyrimidin;
- 2-Amino-4-*n*-propylamino-6-(3,4-dimethoxyphenyl)-pyrido[2,3-d]pyrimidin;
- 2-Amino-4-isopropylamino-6-(3,4-dimethoxyphenyl)-pyrido[2,3-d]pyrimidin;
- 2-Amino-4-(1-piperidino)-6-(3,4-dimethoxyphenyl)-pyrido[2,3-d]pyrimidin;
- 2-Amino-4-(tetrahydro-2H-pyran-4-yloxy)-6-(3,4-dimethoxyphenyl)-pyrido[2,3-d]pyrimidin;
- 2-Amino-6-(3,4-dimethoxyphenyl)-4-(4-*N*-methylpiperidinoxy)-pyrido[2,3-d]pyrimidin;
- 2-Amino-4-methoxy-6-(3,4-dimethoxyphenyl)-pyrido[2,3-d]pyrimidin;
- 2-Pivaloylamino-4-*N*-homopiperazino-6-brom-pyrido[2,3-d]pyrimidin;
- 2-Pivaloylamino-4-*N*-homopiperazino-6-(3,4-dimethoxy-phenyl)-pyrido[2,3-d]pyrimidin;
- 2-Amino-4-*N*-homopiperazino-6-brom-pyrido[2,3-d]pyrimidin;
- 2-Amino-4-*N*-homopiperazino-6-(3,4-dimethoxyphenyl)-pyrido[2,3-d]pyrimidin;
- 2-Amino-4-(sec-butoxy)-6-(3,4-dimethoxyphenyl)-pyrido [2,3-d]pyrimidin;
- 2-Amino-4-(sec-butoxy)-6-(3,4-dimethoxyphenyl)-pyrido[2,3-d]pyrimidin Hydrochloridsalz;
- 2-Amino-4-isopropoxy-6-(3,4-dimethylphenyl)-pyrido[2,3-d]pyrimidin;
- 2-Pivaloylamino-4-(3-brom-anilino)-6-(3,4-dimethoxyphenyl)-pyrido[2,3-d]pyrimidin;
- 2-Pivaloylamino-4-(benzylamino)-6-(3,4-dimethoxyphenyl)-pyrido[2,3-d]pyrimidin;
- 2-Pivaloylamino-4-(phenylethylamino)-6-(3,4-dimethoxyphenyl)-pyrido[2,3-d]pyrimidin;
- 2-Amino-4-(3-brom-anilino)-6-(3,4-dimethoxyphenyl)-pyrido[2,3-d]pyrimidin;
- 2-Amino-4-(benzylamino)-6-(3,4-dimethoxyphenyl)-pyrido[2,3-d]pyrimidin;
- 2-Amino-4-(phenylethylamino)-6-(3,4-dimethoxyphenyl)-pyrido[2,3-d]pyrimidin;
- 2-Pivaloylamino-4-*N*-homopiperazino-6-brom-pyrido[2,3-d]pyrimidin;
- 2-Amino-4-*N*-homopiperazino-6-brom-pyrido[2,3-d]pyrimidin;
- 2-Pivaloylamino-4-*N*-homopiperazino-6-(3,4-dimethoxy-phenyl)-pyrido[2,3-d]pyrimidin;
- 2-Amino-4-*N*-homopiperazino-6-(3,4-dimethoxy-phenyl)-pyrido[2,3-d]pyrimidin;
- 2-Pivaloylamino-6-(4-fluorphenyl)-pyrido[2,3-d]pyrimidin-4-(3H)-on;
- 2-Pivaloylamino-4-(1,2,4-triazolyl)-6-brom-pyrido[2,3-d]pyrimidin;
- 2-Pivaloylamino-4-(3-methylanilino)-6-brom-pyrido[2,3-d]pyrimidin;
- 2-Amino-4-(3-methylanilino)-6-brom-pyrido[2,3-d]pyrimidin;
- 2-Pivaloylamino-4-(*N*-piperidino)-6-(4-fluorphenyl)-pyrido[2,3-d]pyrimidin;
- 2-Pivaloylamino-4-(*N*-morpholino)-6-(4-fluorphenyl)-pyrido[2,3-d]pyrimidin;
- 2-Pivaloylamino-4-(*N*-acetyl-*N*-piperazin-1-yl)-6-(4-fluorphenyl)-pyrido[2,3-d]pyrimidin;
- 2-Pivaloylamino-4-(*N*-methyl-*N*-piperazin-1-yl)-6-(4-fluorphenyl)-pyrido[2,3-d]pyrimidin;
- 2-Amino-4-(*N*-piperidino)-6-(4-fluorphenyl)-pyrido[2,3-d]pyrimidin;
- 2-Amino-4-(*N*-morpholino)-6-(4-fluorphenyl)-pyrido[2,3-d]pyrimidin;
- 2-Amino-4-(*N*-acetyl-*N*-piperazin-1-yl)-6-(4-fluorphenyl)-pyrido[2,3-d]pyrimidin;
- 2-Amino-4-(*N*-methyl-*N*-piperazin-1-yl)-6-(4-fluorphenyl)-pyrido[2,3-d]pyrimidin;
- 2-Pivaloylamino-4-ethoxy-6-(4-fluorphenyl)-pyrido[2,3-d]pyrimidin;
- 2-Pivaloylamino-4-isopropoxy-6-(4-fluorphenyl)-pyrido[2,3-d]pyrimidin;
- 2-Amino-4-ethoxy-6-(4-fluorphenyl)-pyrido[2,3-d]pyrimidin und
- 2-Amino-4-isopropoxy-6-(4-fluorphenyl)-pyrido[2,3-d]pyrimidin.

**12.** Pharmazeutische Zusammensetzung umfassend ein Pyrido(2,3-d)pyrimidinderivat gemäß Anspruch 1.

**13.** Pharmazeutische Zusammensetzung gemäß Anspruch 12, wobei $R_3$ eine monosubstituierte oder polysubstituierte Phenylgruppe ist.

**14.** Pharmazeutische Zusammensetzung gemäß Anspruch 12 oder Anspruch 13, wobei $R_3$ eine Phenylgruppe ist, die nicht mehr als zwei Substituenten hat und wobei ein Substituent paraständig am Phenylring ist.

**15.** Pharmazeutische Zusammensetzung gemäß Anspruch 12, wobei $R_3$ Thienyl ist.

**16.** Pharmazeutische Zusammensetzung gemäß einem der Ansprüche 12 bis 15, ferner umfassend einen oder mehrere pharmazeutisch akzeptable(n) Träger.

**17.** Pharmazeutische Zusammensetzung gemäß einem der Ansprüche 12 bis 16, ferner umfassend einen oder mehrere biologisch aktive(n) Wirkstoff(e), ausgewählt aus der Gruppe bestehend aus immunsupprimierenden und/oder immunmodulatorischen Wirkstoffen und antineoplastischen Wirkstoffen.

**18.** Verfahren zur Herstellung eines Pyrido(2,3-d)pyrimidinderivats gemäß einem der Ansprüche 1 bis 11, umfassend einen Schritt der Umsetzung eines 2-$R_1$-substituierten-6-Brom-pyrido[2,3-d]pyrimidins oder eines 2-$R_1$-substituierten-6-Chlor-pyrido[2,3-d]pyrimidins, wobei $R_1$ wie in der allgemeinen Formel (I) definiert ist, mit einer Arylborsäure, die das Radikal $R_3$ umfasst, wobei $R_3$ eine Aryl- oder Heteroarylgruppe ist, wie in der allgemeinen Formel (I) definiert.

**19.** Verfahren gemäß Anspruch 18, wobei $R_1$ eine Aminogruppe ist und wobei das Verfahren einen Schützungsschritt für die Aminogruppe umfasst.

**20.** Verfahren nach Anspruch 18 oder Anspruch 19, ferner umfassend einen Schritt der Aktivierung einer Hydroxylgruppe in der Position 4 des Pyrido[2,3-d]pyrimidingerüsts.

**21.** Pyrido(2,3-d)pyrimidinderivat gemäß einem der Ansprüche 1 bis 11 zur Verwendung bei der Prävention oder Behandlung von Immunkrankheiten, Autoimmunkrankheiten, kardiovaskulären Krankheiten, Krankheiten des Zentralnervensystems, TNF-alpha-bezogenen Krankheiten und Krankheiten der Zellproliferation.

**Revendications**

**1.** Dérivé de pyrido(2,3-d)pyrimidine de formule générale :

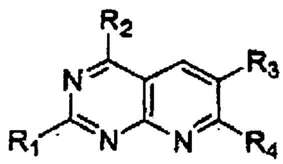

dans laquelle :

$R_1$ est un radical amino,
$R_2$ est choisi dans l'ensemble constitué par les halogènes et les radicaux hydroxy, monoalkylamino en $C_{1-7}$ ; monoarylamino ; monoaryl(alkylamino en $C_{1-7}$) ; morpholinyle ; N-pipéridinyle ; triazolyle ; amino à substitution hétérocyclique ; alcoxy en $C_{1-7}$ ; oxyhétérocycliques ; pipérazinyle ou homopipérazinyle, où ledit radical pipérazinyle est éventuellement N-substitué par un radical acyl (alkyle en $C_{1-7}$) ou arylalkyle ;
$R_3$ est choisi dans l'ensemble constitué par les groupes aryle et hétéroaryle éventuellement substitués par un ou plusieurs substituants choisis dans l'ensemble constitué par les halogènes et les radicaux halogénoalkyle en $C_{1-7}$, alcoxy en $C_{1-7}$ et alkylamino, et
$R_4$ est l'hydrogène,

ou un sel d'addition acceptable en pharmacie de celui-ci,
ou un dérivé dihydro ou un dérivé tétrahydro de celui-ci,
ou une forme isomère stéréochimique de celui-ci, ou un N-oxyde de celui-ci, ou un solvate de celui-ci.

**2.** Dérivé de pyrido(2,3-d)pyrimidine selon la revendication 1, dans lequel $R_2$ est un halogène ou un radical triazolyle.

**3.** Dérivé de pyrido(2,3-d)pyrimidine selon l'une quelconque des revendications 1 à 4, dans lequel $R_3$ est un groupe

phényle monosubstitué ou polysubstitué.

**4.** Dérivé de pyrido(2,3-d)pyrimidine selon l'une quelconque des revendications 1, 2 et 4 à 8, dans lequel $R_3$ est un groupe phényle ayant au plus deux substituants, et dans lequel un substituant est en position para sur ledit cycle phényle.

**5.** Dérivé de pyrido(2,3-d)pyrimidine selon l'une quelconque des revendications 1 à 4, dans lequel $R_3$ est un radical thiényle.

**6.** Dérivé de pyrido(2,3-d)pyrimidine selon l'une quelconque des revendications 1 et 3 à 5, dans lequel $R_2$ est un radical tétrahydropyranyloxy ou méthylpipéridinoxy.

**7.** Dérivé de pyrido(2,3-d)pyrimidine selon l'une quelconque des revendications 1 et 3 à 5, dans lequel $R_2$ est un radical alcoxy en $C_{1-4}$.

**8.** Dérivé de pyrido(2,3-d)pyrimidine selon l'une quelconque des revendications 1 et 3 à 5, dans lequel $R_2$ est un radical monopropylamino, anilino, bromoanilino, benzylamino ou phényléthylamino.

**9.** Dérivé de pyrido(2,3-d)pyrimidine selon l'une quelconque des revendications 1 et 3 à 5, dans lequel $R_2$ est un groupe pipérazin-1-yle, ledit groupe étant éventuellement substitué en position 4 par un substituant $R_5$, lequel $R_5$ est choisi dans l'ensemble constitué par : $COR_8$ où $R_8$ est choisi parmi l'hydrogène et les radicaux alkyle en $C_{1-7}$, aryle éventuellement substitué par un ou plusieurs substituants, aryloxy, et arylamino ; et $R_{11}$, lequel $R_{11}$ est un radical arylalkyle.

**10.** Dérivé de pyrido(2,3-d)pyrimidine selon l'une quelconque des revendications 1 et 3 à 5, dans lequel $R_2$ est un radical méthylphénylcarbamoylpipérazin-1-yle, *N*-phénoxyacétyl-*N*-pipérazin-1-yle, (*N*-benzyloxycarbonyl)-pipérazin-1-yle or *N*-benzylpipérazin-1-yle.

**11.** Dérivé de pyrido(2,3-d)pyrimidine choisi dans l'ensemble constitué par les suivantes :

2-amino-4-isopropoxy-6-bromo-pyrido[2,3-d]pyrimidine,
2-amino-4-isopropoxy-6-(3,4-diméthoxyphényl)-pyrido[2,3-d]pyrimidine,
2-pivaloylamino-4-morpholino-6-bromo-pyrido[2,3-d]-pyrimidine,
2-amino-4-morpholino-6-(3,4-diméthoxyphényl)-pyrido[2,3-d]pyrimidine,
2-pivaloylamino-4-*N*-pipérazino-6-bromo-pyrido[2,3-d]-pyrimidine,
2-pivaloylamino-4-[*N*-4-méthylphénylcarbamoyl-pipérazin-1-yl]-6-bromo-pyrido[2,3-d]pyrimidine,
2-amino-4-[N-4-méthylphénylcarbamoyl-pipérazin-1-yl]-6-bromo-pyrido[2,3-d]pyrimidine,
2-pivaloylamino-4-*N*-pipérazinyl-6-(3,4-diméthoxyphényl)-pyrido[2,3-d]pyrimidine,
2-pivaloylamino-4-[(N-phénoxyacétyl)-N-pipérazin-1-yl]-6-(3,4-diméthoxyphényl)-pyrido[2,3-d]pyrimidine,
2-amino-4-(*N*-phénoxyacétyl-*N*-pipérazin-1-yl)-6-(3,4-diméthoxyphényl)pyrido[2,3-d]pyrimidine,
2-pivaloylamino-4-[N-4-méthylphénylcarbamoyl-pipérazin-1-yl]-6-(3,4-diméthoxy-phényl)pyrido[2,3-d]-pyrimidine,
2-amino-4-(*N*-4-méthylphénylcarbamoyl-pipérazin-1-yl]-6-(3,4-diméthoxyphényl)pyrido[2,3-d]pyrimidine,
2-pivaloylamino-4-[(N-benzyloxycarbonyl)-pipérazin-1-yl]-6-bromo-pyrido[2,3-d]pyrimidine,
2-pivaloylamino-4-[(*N*-benzyloxycarbonyl)-pipérazin-1-yl]-6-(3,4-diméthoxyphényl)-pyrido[2,3-d]pyrimidine,
2-amino-6-(3,4-diméthoxyphényl)-4-[(*N*-benzyloxycarbonyl)-pipérazin-1-yl]-pyrido[2,3-d]pyrimidine,
2-pivaloylamino-4-isopropoxy-6-(4-diméthylaminophényl)pyrido[2,3-d]pyrimidine,
2-pivaloylamino-4-isopropoxy-6-(4-trifluorométhylphényl)pyrido[2,3-d]pyrimidine ;
2-pivaloylamino-4-isopropoxy-6-(2-thiényl)pyrido[2,3-d]pyrimidine,
2-amino-4-isopropoxy-6-(4-diméthylaminophényl)-pyrido[2,3-d]pyrimidine ;
2-amino-4-isopropoxy-6-(4-trifluorométhylphényl)-pyrido[2,3-d]pyrimidine,
2-amino-4-isopropoxy-6-(2-thiényl)pyrido[2,3-d]-pyrimidine,
2-amino-4-(pipérazin-1-yl)-6-(3,4-diméthoxyphényl)-pyrido[2,3-d]pyrimidine,
2-amino-4-(4-benzylpipérazin-1-yl)-6-(3,4-diméthoxyphényl)-pyrido[2,3-d]pyrimidine,
2-pivaloylamino-4-(1,2,4-triazolyl)-6-(3,4-diméthoxyphényl)pyrido[2,3-d]pyrimidine,
2-pivaloylamino-4-n-propylamino-6-(3,4-diméthoxyphényl)pyrido[2,3-d]pyrimidine ;
2-pivaloylamino-4-isopropylamino-6-(3,4-diméthoxyphényl)pyrido[2,3-d]pyrimidine ;
2-pivaloylamino-4-(1-pipéridino)-6-(3,4-diméthoxyphényl)pyrido[2,3-d]pyrimidine ;

2-pivaloylamino-4-(tétrahydro-2H-pyran-4-yloxy)-6-(3,4-diméthoxyphényl)pyrido[2,3-d]pyrimidine,
2-pivaloylamino-6-(3,4-diméthoxyphényl)-4-(4-N-méthylpipéridinoxy)pyrido[2,3-d]pyrimidine ;
2-amino-4-*n*-propylamino-6-(3,4-diméthoxyphényl)pyrido[2,3-d]pyrimidine ;
2-amino-4-isopropylamino-6-(3,4-diméthoxyphényl)pyrido[2,3-d]pyrimidine ;
2-amino-4-(1-pipéridino)-6-(3,4-diméthoxyphényl)-pyrido[2,3-d]pyrimidine ;
2-amino-4-(tétrahydro-2H-pyran-4-yloxy)-6-(3,4-diméthoxyphényl)pyrido[2,3-d]pyrimidine ;
2-amino-6-(3,4-diméthoxyphényl)-4-(4-*N*-méthylpipéridinoxy)pyrido[2,3-d]pyrimidine ;
2-amino-4-méthoxy-6-(3,4-diméthoxyphényl)pyrido[2,3-d]pyrimidine ;
2-pivaloylamino-4-*N*-homopipérazino-6-bromopyrido[2,3-d]pyrimidine ;
2-pivaloylamino-4-*N*-homopipérazino-6-(3,4-diméthoxyphényl)-pyrido[2,3-d]pyrimidine ;
2-amino-4-*N*-homopipérazino-6-bromo-pyrido[2,3-d]-pyrimidine ;
2-amino-4-*N*-homopipérazino-6-(3,4-diméthoxyphényl)-pyrido[2,3-d]pyrimidine ;
2-amino-4-(sec-butoxy)-6-(3,4-diméthoxyphényl)-pyrido[2,3-d]pyrimidine ;
sel chlorhydrate de 2-amino-4-(sec-butoxy)-6-(3,4-diméthoxyphényl)-pyrido[2,3-d]pyrimidine ;
2-amino-4-isopropoxy-6-(3,4-diméthylphényl)-pyrido[2,3-d]pyrimidine ;
2-pivaloylamino-4-(3-bromo-anilino)-6-(3,4-diméthoxyphényl)pyrido[2,3-d]pyrimidine ;
2-pivaloylamino-4-(benzylamino)-6-(3,4-diméthoxyphényl)pyrido[2,3-d]pyrimidine ;
2-pivaloylamino-4-(phényléthylamino)-6-(3,4-diméthoxyphényl)pyrido[2,3-d]pyrimidine ;
2-amino-4-(3-bromo-anilino)-6-(3,4-diméthoxyphényl)-pyrido[2,3-d]pyrimidine ;
2-amino-4-(benzylamino)-6-(3,4-diméthoxyphényl)-pyrido[2,3-d]pyrimidine ;
2-amino-4-(phényléthylamino)-6-(3,4-diméthoxyphényl)-pyrido[2,3-d]pyrimidine ;
2-pivaloylamino-4-*N*-homopipérazino-6-bromopyrido[2,3-d]pyrimidine ;
2-amino-4-*N*-homopipérazino-6-bromo-pyrido[2,3-d]-pyrimidine ;
2-pivaloylamino-4-*N*-homopipérazino-6-(3,4-diméthoxyphényl)-pyrido[2,3-d]pyrimidine ;
2-amino-4-*N*-homopipérazino-6-(3,4-diméthoxy-phényl)-pyrido[2,3-d]pyrimidine ;
2-pivaloylamino-6-(4-fluorophényl)pyrido[2,3-d]-pyrimidin-4-(3H)-one ;
2-pivaloylamino-4-(1,2,4-triazolyl)-6-bromopyrido[2,3-d]pyrimidine ;
2-pivaloylamino-4-(3-méthylanilino)-6-bromopyrido[2,3-d]pyrimidine ;
2-amino-4-(3-méthylanilino)-6-bromo-pyrido[2,3-d]-pyrimidine ;
2-pivaloylamino-4-(*N*-pipéridino)-6-(4-fluorophényl)-pyrido[2,3-d]pyrimidine ;
2-pivaloylamino-4-(*N*-morpholino)-6-(4-fluorophényl)-pyrido[2,3-d]pyrimidine ;
2-pivaloylamino-4-(*N*-acétyl-*N*-pipérazin-1-yl)-6-(4-fluorophényl)pyrido[2,3-d]pyrimidine ;
2-pivaloylamino-4-(*N*-méthyl-*N*-pipérazin-1-yl)-6-(4-fluorophényl)pyrido[2,3-d]pyrimidine ;
2-amino-4-(*N*-pipéridino)-6-(4-fluorophényl)-pyrido[2,3-d]pyrimidine ;
2-amino-4-(*N*-morpholino)-6-(4-fluorophényl)-pyrido[2,3-d]pyrimidine ;
2-amino-4-(*N*-acétyl-*N*-pipérazin-1-yl)-6-(4-fluorophényl)pyrido[2,3-d]pyrimidine ;
2-amino-4-(*N*-méthyl-*N*-pipérazin-1-yl)-6-(4-fluorophényl)pyrido[2,3-d]pyrimidine ;
2-pivaloylamino-4-éthoxy-6-(4-fluorophényl)-pyrido[2,3-d]pyrimidine ;
2-pivaloylamino-4-isopropoxy-6-(4-fluorophényl)-pyrido[2,3-d]pyrimidine ;
2-amino-4-éthoxy-6-(4-fluorophényl)pyrido[2,3-d]-pyrimidine ; et
2-amino-4-isopropoxy-6-(4-fluorophényl)pyrido[2,3-d]-pyrimidine.

**12.** Composition pharmaceutique comprenant un dérivé de pyrido(2,3-d)pyrimidine selon la revendication 1.

**13.** Composition pharmaceutique selon la revendication 12, dans laquelle $R_3$ est un groupe phényle monosubstitué ou polysubstitué.

**14.** Composition pharmaceutique selon la revendication 12 ou la revendication 13, dans laquelle $R_3$ est un groupe phényle ayant au plus deux substituants, et dans laquelle un substituant est en position para sur ledit cycle phényle.

**15.** Composition pharmaceutique selon la revendication 12, dans laquelle $R_3$ est un radical thiényle.

**16.** Composition pharmaceutique selon l'une quelconque des revendications 12 à 15, comprenant en outre un ou plusieurs véhicules acceptables en pharmacie.

**17.** Composition pharmaceutique selon l'une quelconque des revendications 12 à 16, comprenant en outre un ou plusieurs médicaments biologiquement actifs, choisis dans l'ensemble constitué par les médicaments immunodépresseurs et/ou immunomodulateurs, et les médicaments antinéoplasiques.

**18.** Procédé pour préparer un dérivé de pyrido(2,3-d)pyrimidine selon l'une quelconque des revendications 1 à 11, comprenant une étape consistant à faire réagir une 6-bromopyrido[2,3-d]pyrimidine 2-$R_1$-substituée ou une 6-chloropyrido[2,3-d]pyrimidine 2-$R_1$-substituée, où $R_1$ est tel que défini dans la formule générale (I), avec un acide arylboronique contenant le radical $R_3$, où $R_3$ est un groupe aryle ou hétéroaryle tel que défini dans la formule générale (I).

**19.** Procédé selon la revendication 18, dans lequel $R_1$ est un groupe amino, et lequel procédé comprend une étape de protection pour ledit groupe amino.

**20.** Procédé selon la revendication 18 ou la revendication 19, comprenant en outre une étape consistant à activer un groupe hydroxyle en position 4 de la charpente de pyrido[2,3-d]pyrimidine.

**21.** Dérivé de pyrido(2,3-d)pyrimidine selon l'une quelconque des revendications 1 à 11, pour une utilisation dans la prévention ou le traitement de troubles immuns, de troubles auto-immuns, de troubles cardiovasculaires, de troubles du système nerveux central, de troubles liés au TNF-alpha et de troubles de prolifération cellulaire.

Figure 1

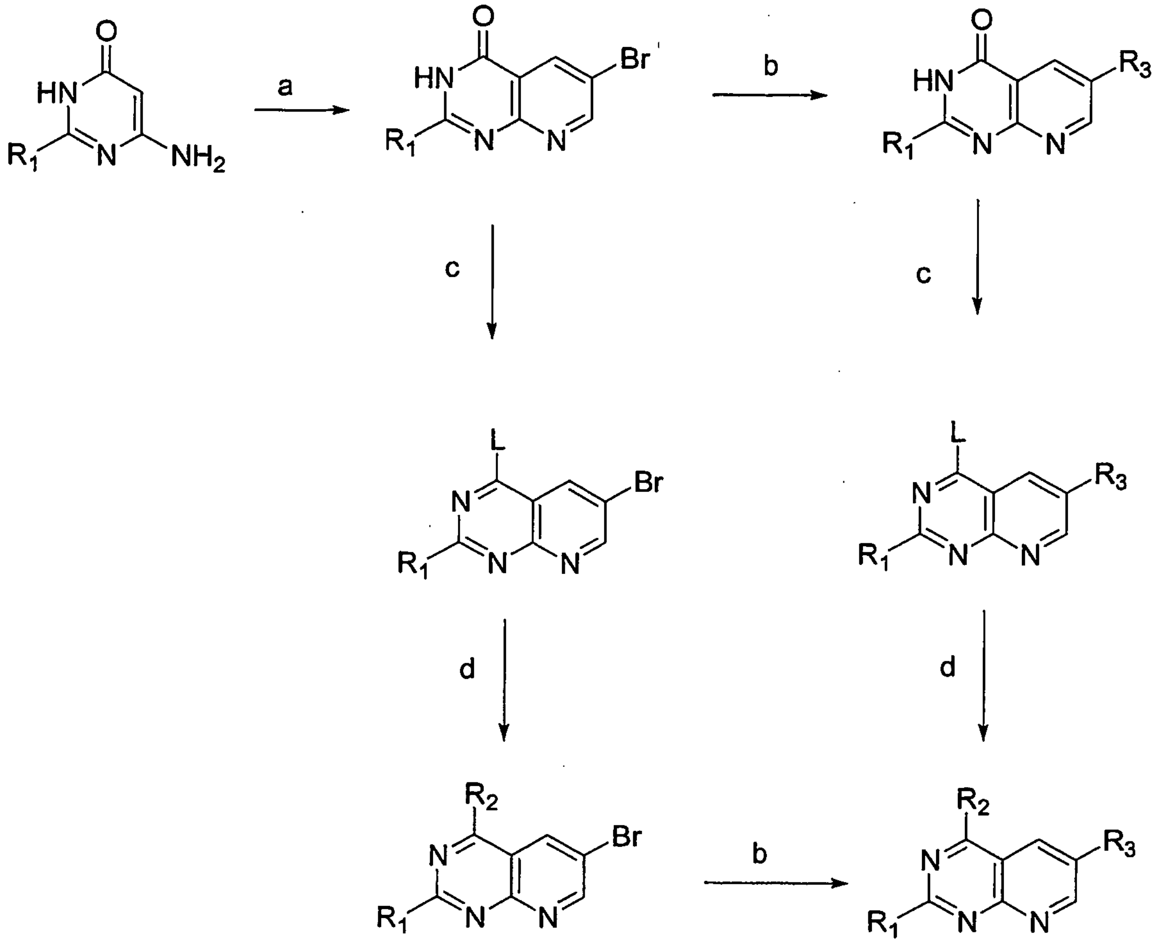

Figure 2

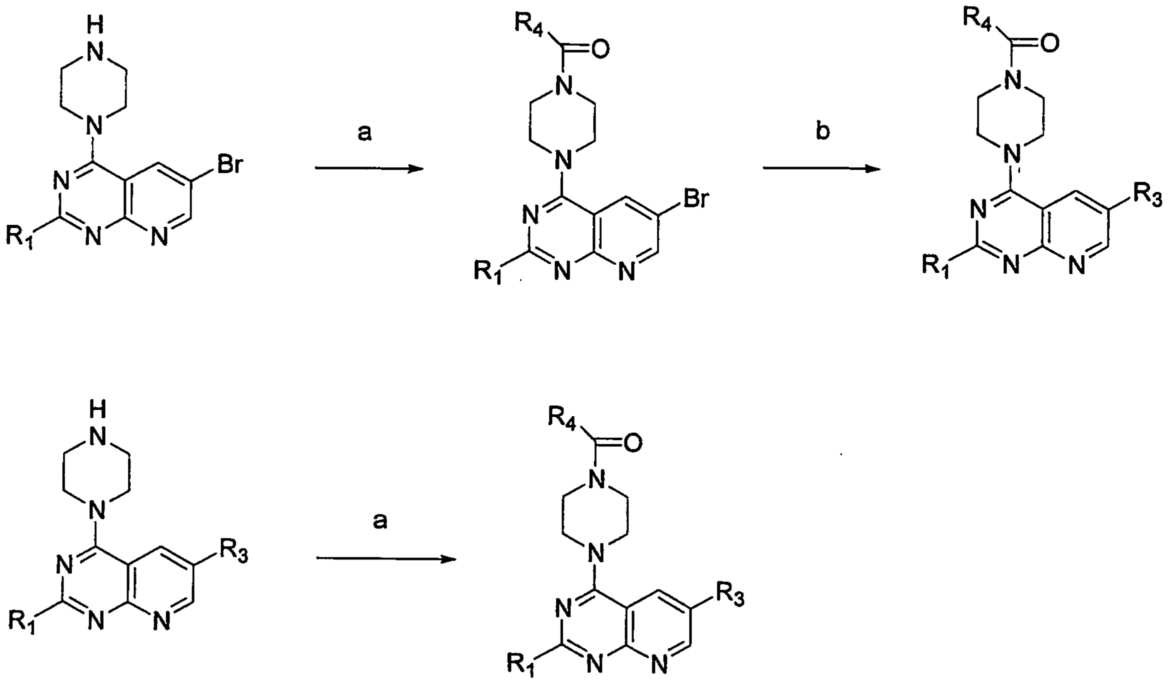

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5223503 A
- US 5547954 A
- US 2512572 A
- US 5665772 A
- US 5780462 A
- US 6440991 B
- US 6331547 B


### Non-patent literature cited in the description


- **TAYLOR et al.** *Heteocycles,* 1993, vol. 36, 1885
- **DURUCASU.** *Turk. J. Chem.,* 1989, vol. 13, 280-292
- **NENOVA et al.** *Archives of Hellenic Medicine,* 2000, vol. 17, 619-621
- **SAARINEN et al.** *Cancer Research,* 1990, vol. 50, 592-595
- **TAYLOR et al.** *Synth. Commun,* 1988, vol. 18, 1187-1191
- **CHOU et al.** *Adv. Enzyme Reg.,* 1984, vol. 22, 27
- **LIN et al.** *Transplantation,* 1997, vol. 63, 1734-1738
- McCutcheon's Detergents and Emulsifiers Annual. MC Publishing Crop, 1981
- Tensid-Taschenbuch. Hanser Verlag, 1981
- Encyclopaedia of Surfactants. Chemical Publishing Co, 1981
- **TAYLOR et al.** *Heterocycles,* 1993, vol. 36, 1889
- **TAYLOR et al.** *Heterocycles,* 1993, vol. 36, 1883
- **TAYLOR et al.** *Hetercycles,* 1993, vol. 36, 1883